# EUROPEAN PATENT APPLICATION

(11) **EP 3 680 250 A1**
(43) Date of publication of application: **15.07.2020**
(21) Application number: 18854495.1
(22) Date of filing: 07.09.2018
(51) Int. Cl.: C07K 7/08, A61K 45/06, A61K 47/68, A61K 38/00

(54) **COMPOSITION FOR RESTRAINING ACTIVITY OF REGULATORY T CELL, COMPRISING PEPTIDE BINDING SPECIFICALLY TO NEUROPILIN 1 (NRP1)**

(30) Priority: 08.09.2017 KR 20170115262
(71) Applicant: Ajou University Industry-Academic Cooperation Foundation, Gyeonggi-do 16499 (KR)
(72) Inventor: KIM, Yong Sung, Suwon-si Gyeonggi-do 16534 (KR); KIM, Ye Jin, Busan 49497 (KR); JUNG, Keunok, Suwon-si Gyeonggi-do 16532 (KR); KIM, Jeong-Ah, Gwangju-si Gyeonggi-do 12791 (KR)
(74) Representative: dompatent von Kreisler Selting Werner - Partnerschaft von Patent- und Rechtsanwälten mbB
(86) International application number: PCT/KR2018/010487
(87) International publication number: WO 2019/050326

(57) **Abstract**

The present invention relates to a composition for inhibiting regulatory T cell immunosuppression, containing a fusion protein comprising a peptide that binds specifically to neuropilin 1 (NRP1). The fusion protein comprising the peptide that binds specifically to neuropilin 1 according to the present invention can reduce the proportion of regulatory T cells among CD4+ T cells in tumor tissue and inhibit the activity of regulatory T cells that infiltrated into tumor tissue, thereby down-regulating immunosuppression in the tumor tissue without causing systemic toxicity. Thus, the fusion protein can exhibit a high effect on tumor treatment.

## Description

### TECHNICAL FIELD

The present invention relates to a composition for inhibiting regulatory T cell (Treg) activity, comprising a peptide that binds specifically to neuropilin 1 (NRP1), and more particularly to a composition for inhibiting immunosuppression by inhibiting regulatory T cell activity in tumor tissue. (Moreover, the present invention relates to a composition for inhibiting cancer cell growth by inhibiting the activity of regulatory T cells in tumor tissue to increase the growth of cytotoxic T cells in the tumor tissue.

In addition, the present invention relates to a composition which inhibits cancer cell growth by targeting neuropilin 1 overexpressed in regulatory T cells in tumor tissue and inhibiting the activity of the regulatory T cells, but does not show systemic toxicity resulting from inhibition of the activity of the regulatory T cells.

### BACKGROUND ART

The immune system is a very important system that recognizes infection and protects our body by humoral and cell-mediated responses. If the immune system is impaired, infectious diseases occur and the function of suppressing cancer also decreases. T cells play an important role in controlling the entire immune response, and among T cells, regulatory T cells (Tregs) are essential for suppressing autoimmune diseases and maintaining the homeostasis of immune function. However, regulatory T cells in tumor microenvironments promote the progression of diseases such as cancer by reducing the number of immunocytes such as cytotoxic T lymphocytes (CTLs) and natural killer cells (NK cells) and suppressing their function.

It is known that regulatory T cells in tumor microenvironments perform immunosuppression through the following four mechanisms. First, regulatory T cells secrete inhibitory cytokines, such as interleukin-10, TGF-β and interleukin-35, which act on various innate immune cells and effector T cells in close proximity to suppress their anticancer immune response (Nakamura et *al.,* 2001; Collison et *al.,* 2007). Second, it is known that CD25 (interleukin-2 receptor alpha-chain, IL-2Rα chain), which is expressed at high levels on the surface of regulatory T cells, results in the removal of interleukin-2 distributed locally around the cells, which suppresses effector T cell activation due to cytokine deficiency and induces apoptosis (Thornton and Shevach, 1998). The third mechanism is a mechanism that induces intracellular or extracellular release of adenosine nucleosides, and in the former case, regulatory T cells perform immunosuppression by delivering the inhibitory signaling substance cAMP (cyclic AMP) directly through gap junctions with effector T cells (Kobie *et al.,* 2006), and in the latter case, pericellular adenosine is produced by the external enzymes CD39 and CD73 present in the cell membrane of regulatory T cells, and is recognized through adenosine receptor 2A (A2AR) present in effector T cells, thereby suppressing the function of effector T cells (Zarek *et al.,* 2008). Finally, there is an inhibitory mechanism on antigen presenting cells, including dendritic cells. The co-stimulatory molecule CTLA-4 (cytotoxic T-lymphocyte associated molecule-4) is expressed on the surface of regulatory T cells, and functions to convert dendritic cells into tolerance-inducing dendritic cells by interaction with CD80/CD86 present on the surface of the dendritic cells. Also, it inhibits the immune function of effector T cells by secreting IDO (indoleamine 2,3-dioxygenase), an enzyme involved in tryptophan metabolism in these dendritic cells (Read *et al.,* 2000; Fallarino *et al.,* 2003). Additionally, the inhibitory mechanism also occurs through lymphocyte-activation gene-3 (LAG-3) present in regulatory T cells. LAG-3 binds strongly to major histocompatibility complex class II (MHC class II) molecules on the surface of immature dendritic cells and inhibits the maturation and immunostimulatory activity of the dendritic cells (Huang *et al*., 2004).

According to these mechanisms, regulatory T cells suppress anticancer immune responses and promote the progression of cancer, and thus therapeutic strategies for increasing anticancer effects by reducing the number of regulatory T cells in tumor tissue have been developed. Until now, there have been developed a method of administering a low concentration of cyclophosphamide (CP), which is a chemotherapy that modulates immunosuppression, and methods of using antibodies that target molecules, such as CD25, CTLA-4 and PD-1, and chemokine receptors, which are expressed specifically by regulatory T cells. In particular, antibodies approved for use in clinical trials by the US Food and Drug Administration include daclilizumab that targets CD25 expressed in regulatory T cells, and ipilimumab and nivolumab which target CTLA-4 and PD-1 (programmed cell death protein-1), respectively. In addition, antibodies, which target immune checkpoint molecules (LAG-3, TIM-3, GITR, and OX40) and the chemokine receptor CCR4 and reduce the number of regulatory T cells through antibody-dependent cell-mediated cytotoxicity (ADCC), are also under clinical trials. These antibodies that target regulatory T cells are therapeutic strategies for reducing the number of regulatory T cells, but may also cause life-threatening autoimmune reactions or immune-related adverse events (IRAEs) in some patients. Therefore, there is a need for the development of an effective therapy that can block the immunosuppressive function of regulatory T cells in tumor microenvironments while minimizing side effects.

Meanwhile, neuropilin 1 (NRP1), a transmembrane glycoprotein, binds to ligands of the VEGF family and ligands of the semaphorin family (Guo and Vander Kooi, 2015; Prud'homme and Glinka, 2012). It is known that neuropilin 1 is very weakly expressed in normal cells, but is overexpressed in most tumor vascular endothelial cells, solid cancer cells, blood tumor cells, and the like. In addition, it is known that neuropilin 1 is highly expressed on the surface of regulatory T cells in mouse models, and thus is a marker of mouse regulatory T cells (Bruder *et al.,* 2004; Loser *et al*., 2005). In particular, it is known that neuropilin 1 expressed in regulatory T cells interacts with semaphorin-4A (Sema4A) expressed in CD4⁺ T cells or CD8⁺ T cells, and increases regulatory T cell activity by the following mechanisms in the immunosuppressive synapse (Delgoffe *et al*., 2013). Neuropilin 1 plays a role in maintaining the stability and immunosuppressive function of regulatory T cells by: 1) introducing phosphatase and tensin homolog (PTEN); 2) inhibiting Akt signal activation; and 3) maintaining Foxp3 expression by increasing the intranuclear migration of foxo3a, a transcription factor important for Foxp3 expression. Based on this role of neuropilin 1 expressed in regulatory T cells, when the expression of neuropilin 1 in regulatory T cells is decreased, interferon-γ (IFN-γ) secretion and interferon-γ receptor expression in the regulatory T cells are increased, so that the fragility of the regulatory T cells is induced and the inhibitory activity of the regulatory T cells against effector T cells is decreased (Overacre-Delgoffe *et al*., 2017). In addition, it was confirmed that the tumorigenesis of malignant melanoma was relatively suppressed in mice having regulatory T cells lacking neuropilin 1 compared to mice having regulatory T cells in which neuropilin 1 was expressed (Hansen *et al*., 2012).

On the other hand, unlike the mouse model, in humans, the expression of neuropilin 1 in regulatory T cells present in peripheral blood of normal people is low, and there are few reports of this fact. However, it was reported that, unlike regulatory T cells of normal people, about 20 to 30% of regulatory T cells in peripheral blood of melanoma patients and head and neck squamous cell carcinoma patients expressed neuropilin 1, and especially, about 50% or more of regulatory T cells that infiltrated the tumors of these patients expressed neuropilin 1 (Overacre-Delgoffe *et al.,* 2017). In addition, melanoma patients and head and neck squamous cell carcinoma patients with regulatory T cells that express more neuropilin 1 showed a shorter disease-free survival period than patients with regulatory T cells that express less neuropilin 1. This shows that the expression level of neuropilin 1 in regulatory T cells that infiltrated tumors is closely related to the patient's prognosis.

Therefore, a strategy that targets neuropilin 1 expressed in regulatory T cells in human models, can specifically target only regulatory T cells in tumor tissue, unlike a conventional strategy that targets CTLA-4 and the like in regulatory T cells, and thus it can be an effective anticancer therapeutic strategy that can minimize immune-related side effects. To date, the importance against neuropilin 1 expressed in regulatory T cells in mouse models has been studied, but the development of an antibody or anticancer agent that targets neuropilin 1 in regulatory T cells, developed based on these studies, has not been reported yet.

Under this technical background, the present inventors have found that a peptide specific for neuropilin 1 can reduce the activity of regulatory T cells that suppress immune response, thereby inhibiting the immunosuppression.

In particular, the present inventors have found that the tumor growth is inhibited *in vivo* in mice having regulatory T cells through the inhibition of regulatory T cell activity and the increase in the growth of cytotoxic T cells by a peptide specific for neuropilin 1, thereby completing the present invention.

### DISCLOSURE OF INVENTION

### TECHNICAL PROBLEM

It is an object of the present invention to provide a composition containing a peptide that binds specifically to neuropilin 1, in which the composition is capable of inhibiting the activity of regulatory T cells that suppress immune response, thereby down-regulating the activity thereof, and a method for inhibiting the activity of regulatory T cells that suppress immune response, thereby down-regulating the activity thereof.

Another object of the present invention is to provide a composition for inhibiting immunosuppression by inhibiting regulatory T cell activity, the composition containing a peptide that binds specifically to neuropilin 1, and a method for inhibiting immunosuppressive response using a peptide that binds specifically to neuropilin 1.

Still another object of the present invention is to provide an immunomodulating agent, an anticancer agent and an infectious disease therapeutic agent, which comprise a composition containing a peptide that binds specifically to neuropilin 1, in which the composition is capable of inhibiting the activity of regulatory T cells that suppress immune response, thereby down-regulating the activity thereof, and a method for treating the diseases using the same.

Yet another object of the present invention is to provide a composition and a method which inhibit cancer cell growth through the increase in the growth of cytotoxic T cells by targeting neuropilin 1 overexpressed in regulatory T cells in tumor tissue and inhibiting the activity of the regulatory T cells, but do not show systemic toxicity resulting from inhibition of the activity of the regulatory T cells.

### TECHNICAL SOLUTION

To achieve the above object, the present invention provides a composition and method for inhibiting regulatory T cell (Treg) activity, which contains, as an active ingredient, a peptide that binds specifically to neuropilin 1 (NRP1), and a composition for inhibiting immunosuppression by inhibiting regulatory T cell activity and a method for inhibiting immunosuppressive response using the same. The inhibition of immunosuppression can ultimately enhance immunity of cytotoxic T cells in tumor tissue and other immunocytes, thereby treating infectious disease and immune disease, which may be caused by immunosuppression.

The present invention is also directed to a composition that targets neuropilin 1 overexpressed in regulatory T cells that infiltrated into tumor tissue, thereby inhibiting the activity of the regulatory T cells in the tumor tissue, and has only a minimum effect on systemic regulatory T cells so that it does not show systemic toxicity caused by inhibition of regulatory T cell activity, and to a method for inhibiting the activity of the regulatory T cells in the tumor tissue.

The present invention also provides a method for inhibiting the activity of regulatory T cells, which comprises a step of administering the composition to a subject.

The present invention also provides an immunomodulating agent, an infectious disease therapeutic agent and an anticancer agent, which comprise the composition.

The present invention also provides an immunomodulating method, which comprises a step of administering the composition to a subject.

The present invention also provides the use of the composition, for immunomodulation.

The present invention also provides the use of the composition, for the manufacture of a medicament for immunomodulation.

The present invention also provides a method for preventing or treating cancer or infectious disease, which comprises a step of administering the composition to a subject.

The present invention also provides the use of the composition, for the prevention or treatment of cancer or infectious disease.

The present invention also provides the use of the composition, for the manufacture of a medicament for preventing or treating cancer or infectious disease.

The present invention also provides the use of a peptide binding specifically to neuropilin 1, for the inhibition of regulatory T cell (Treg) activity.

### BRIEF DESCRIPTION OF THE DRAWINGS

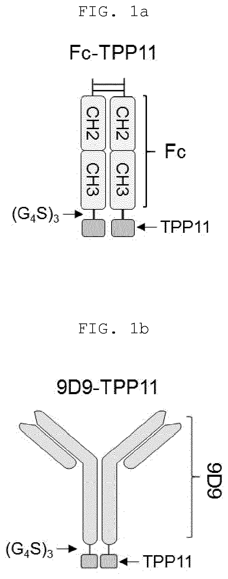

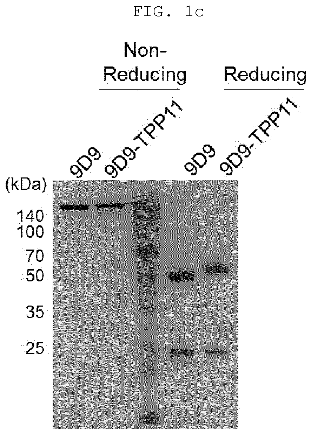

FIG. 1 shows schematic views of constructed Fc-TPP11 and 9D9-TPP11. Specifically, FIG. 1a is a schematic view of Fc-TPP11 in which a TPP11 peptide is fused to the C-terminus of an antibody heavy-chain constant region via a 15-residue (G₄S)₃ linker; FIG. 1b is a schematic view of 9D9-TPP11 in which a TPP11 peptide is fused via a 15-residue (G₄S)₃ linker to the C-terminus of 9D9 which is an anti-mouse CTLA-4 (cytotoxic T-lymphocyte-associated protein 4) antibody; and FIG. 1c shows the results obtained by expressing and purifying wild-type antibody 9D9 and 9D9-TPP11 in animal cells (HEK293F), and then separating 4 µg protein on SDS-PAGE under non-reducing conditions, and analyzing the size and combination form of the protein by Coomassie blue staining.

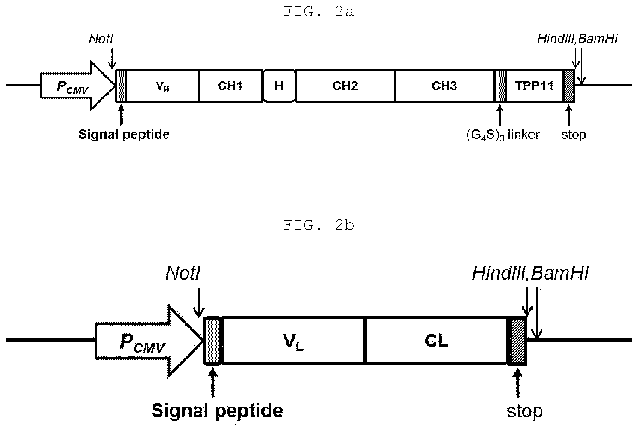

FIG. 2 shows schematic views of vectors for expressing in HEK293F animal cells a 9D9-TPP11 protein in which TPP11 is fused to the heavy-chain constant region of the antibody 9D9. Specifically, FIG. 2a is a schematic view of a 9D9-TPP11 heavy-chain expression vector in which TPP11 is fused to the heavy-chain constant region of 9D9; and FIG. 2b is a schematic view of a 9D9 light-chain expression vector.

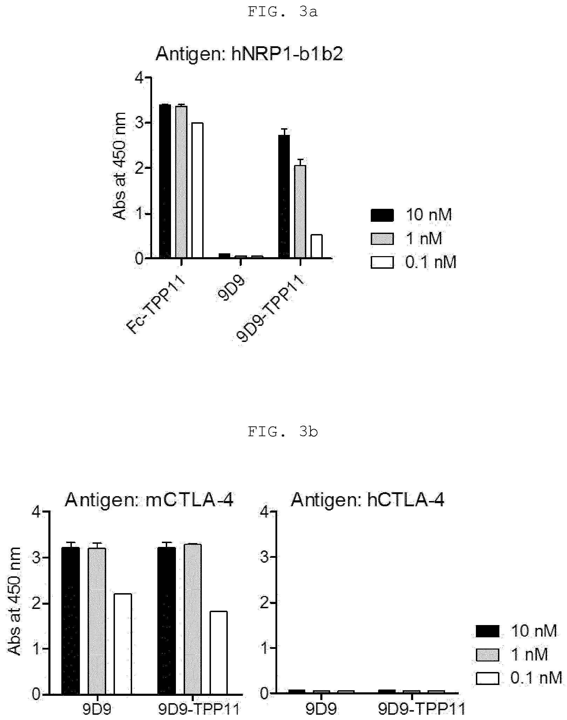

FIG. 3 shows the results of examining the binding affinities of constructed Fc-TPP11 and 9D9-TPP11 for neuropilin 1 (NRP1) and CTLA-4. Specifically, FIG. 3a shows the results of ELISA (enzyme-linked immunosorbent assay) performed to examine whether constructed 9D9-TPP11 shows binding affinity for a neuropilin 1-b1b2 domain (human NRP1-blb2, hNRP1-b1b2), like Fc-TPP11, in comparison with 9D9, and FIG. 3b shows the results of ELISA performed to examine whether constructed 9D9-TPP11 shows binding affinity for mouse CTLA-4 antigen and human CTLA-4 antigen in the same manner as the wild-type antibody 9D9 that shows binding affinity for CTLA-4.

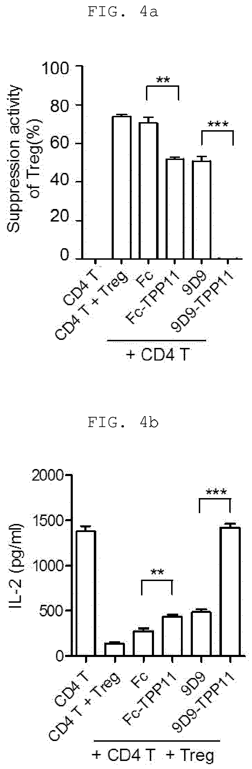

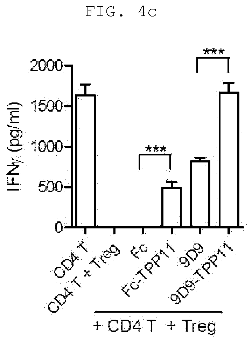

FIG. 4 shows the results of evaluating the regulatory T cell activity inhibitory ability of Fc-TPP11 and 9D9-TPP11 *in vitro.* Specifically, FIG. 4a shows the results of analysis performed to measure the effects of Fc-TPP11 and 9D9-TPP11 against the activity of regulatory T cells that inhibit the growth of CD4⁺ T cells; FIG. 4b shows the results of ELISA performed to measure the secretion of the cytokine interleukin-2 (IL-2) during the growth of CD4⁺ T cells in order to compare the inhibitory effects of Fc-TPP11 and 9D9-TPP11 against regulatory T cell activity; and FIG. 4c shows the results of ELISA performed to measure the secretion of another cytokine interleukin-γ (IL-γ) during the growth of CD4⁺ T cells in order to compare the inhibitory effects of Fc-TPP11 and 9D9-TPP11 against regulatory T cell activity.

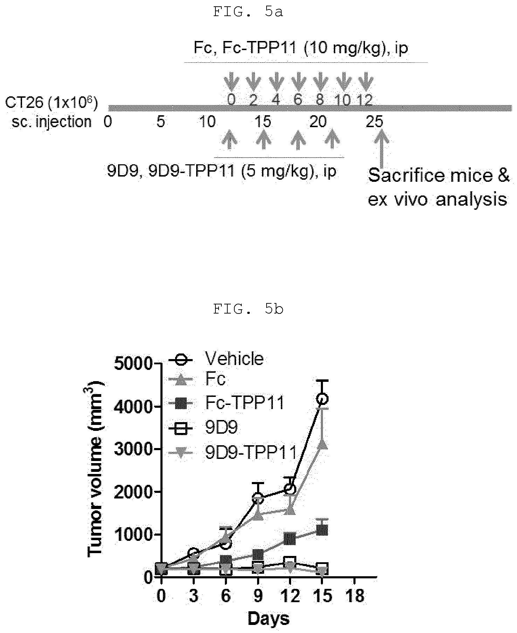

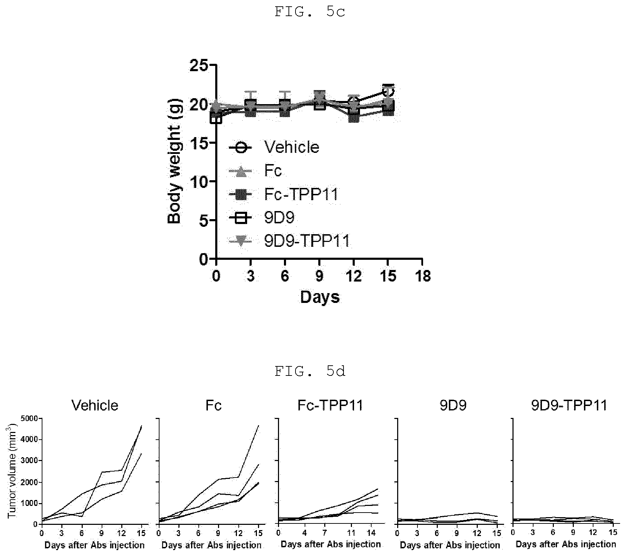

FIG. 5 shows the results of measuring the tumor growth inhibitory activities of Fc-TPP11 and 9D9-TPP11 *in vivo* in BALB/c mice. Specifically, FIG. 5a is a schedule showing the date of tumor transplantation and the administration interval, dose and administration method of drugs in an experiment on tumor growth inhibitory activity *in vivo* in mice; FIGS. 5b and 5c are graphs showing the changes in tumor volume by administration of Fc, Fc-TPP11, 9D9 and 9D9-TPP11 in mice allografted with CT26 mouse colorectal cancer cells (FIG. 5b) and changes in mouse body weight periodically measured during the experiment (FIG. 5c) ; and FIG. 5d is a graph showing the change in tumor volume of each mouse of each administration group as a function of days in the results shown in FIG. 5b.

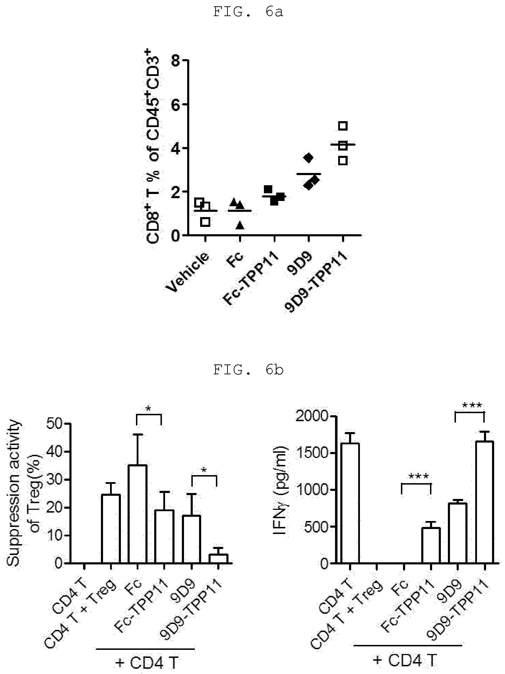

FIG. 6 shows the results obtained by sacrificing mice at 24 hours after the last administration shown in FIG. 5, separating lymphocytes and tumor-infiltrating lymphocytes from the spleens, and evaluating cell activity *in vitro* using the isolated cells. Specifically, FIG. 6a is a graph showing the results of flow cytometry (FACS) performed to analyze the proportion of cytotoxic T cells (CD8⁺) among the tumor-infiltrating lymphocytes obtained by sacrificing mice of each administration group; and FIG. 6b depicts graphs showing the results of evaluating regulatory T cell inhibitory effects by co-culturing the regulatory T cells (CD4⁺CD25⁺) isolated from the tumor-infiltrating lymphocytes of FIG. 6a and the CD4⁺ T cells isolated from the spleen of non-tumor-grafted mice, and measuring the concentration of interferon-gamma secreted by CD4⁺ T cells.

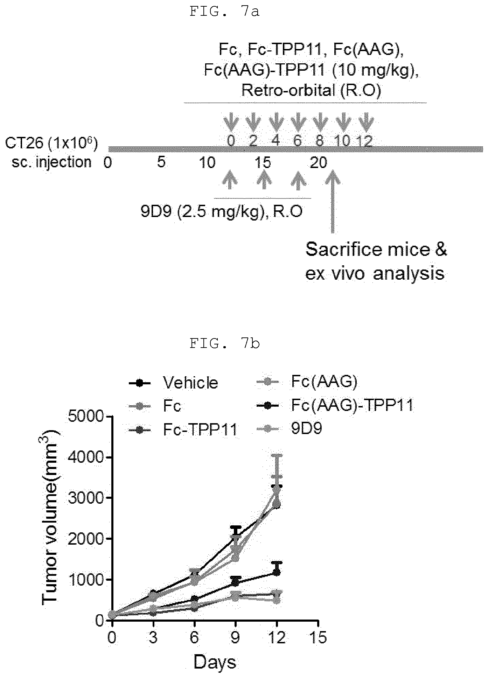

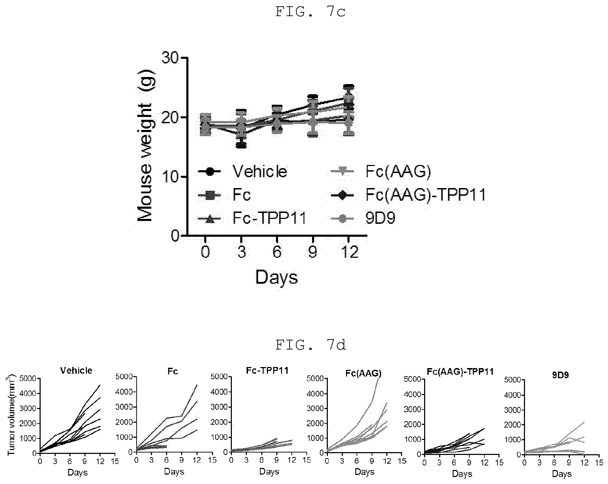

FIG. 7 shows the results of measuring the tumor growth inhibitory activities of Fc-TPP11, Fc(AAG)-TPP11 and 9D9 *in vivo* in BALB/c mice. Specifically, FIG. 7a is a schedule showing the date of tumor transplantation and the administration interval, dose and administration method of drugs in an experiment on tumor growth inhibitory activity *in vivo* in mice; FIGS. 7b and 7c are graphs showing the changes in tumor volume by administration of Fc, Fc-TPP11, 9D9 and 9D9-TPP11 in mice allografted with CT26 mouse colorectal cancer cells (FIG. 7b) and changes in mouse body weight periodically measured during the experiment (FIG. 7c); and FIG. 7d is a graph showing the change in tumor volume of each mouse of each administration group as a function of days in the results shown in FIG. 7b.

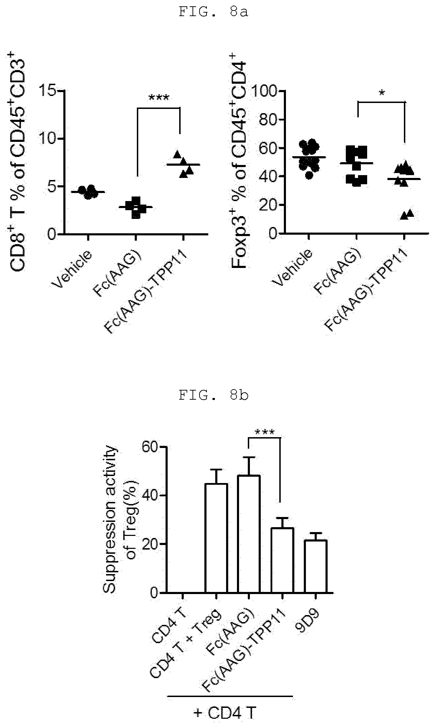

FIG. 8 shows the results obtained by measuring the proportion of tumor-infiltrating lymphocytes obtained by sacrificing mice at 24 hours after the fifth administration of Fc(AAG) and Fc(AAG)-TPP11 in FIG. 7, and evaluating cell activity *in vitro.* Specifically, FIG. 8a is a graph showing the results of flow cytometry (FACS) performed to analyze the proportions of cytotoxic T cells (CD45⁺CD3⁺CD8⁺) and regulatory T cells (CD45⁺CD4⁺Foxp3⁺) among the tumor-infiltrating lymphocytes (CD45⁺) isolated from the sacrificed mice of each administration group; and FIG. 8b is a graph showing the results of evaluating regulatory T cell inhibitory effects by culturing the regulatory T cells (CD4⁺CD25⁺) isolated from the tumor-infiltrating lymphocytes of each administration group and the CD4⁺ T cells (CD4⁺CD25⁻) isolated from the spleen of non-tumor-grafted mice.

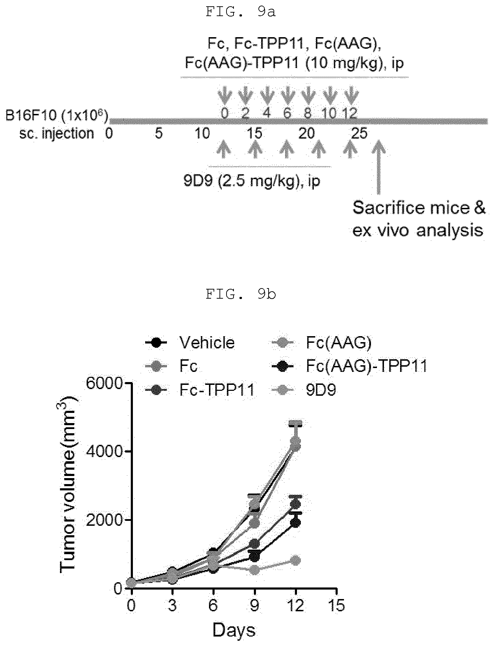

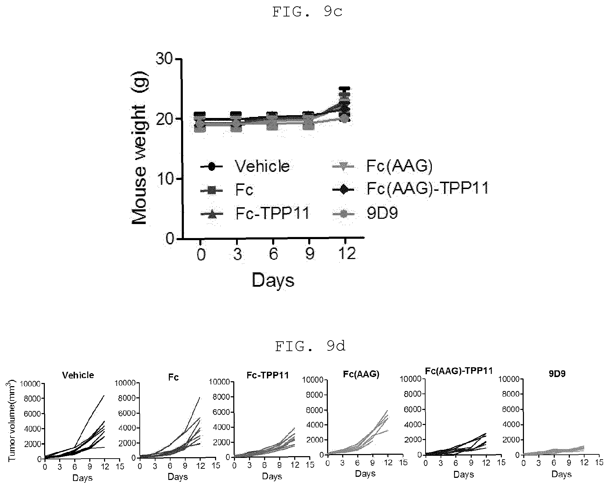

FIG. 9 shows the results of measuring the tumor growth inhibitory activities of Fc-TPP11, Fc(AAG)-TPP11 and 9D9 *in vivo* in C57BL/6 mice. Specifically, FIG. 9a is a schedule showing the date of tumor transplantation and the administration interval, dose and administration method of drugs in an experiment on tumor growth inhibitory activity *in vivo* in mice; FIGS. 9b and 9c are graphs showing the changes in tumor volume by administration of Fc, Fc-TPP11, Fc(AAG), Fc(AAG)-TPP11 and 9D9 in mice allografted with B16F10 mouse malignant melanoma cells (FIG. 9b) and the changes in mouse body weight periodically measured during the experiment (FIG. 9c); and FIG. 9d is a graph showing the change in tumor volume of each mouse of each administration group as a function of days in the results shown in FIG. 9b.

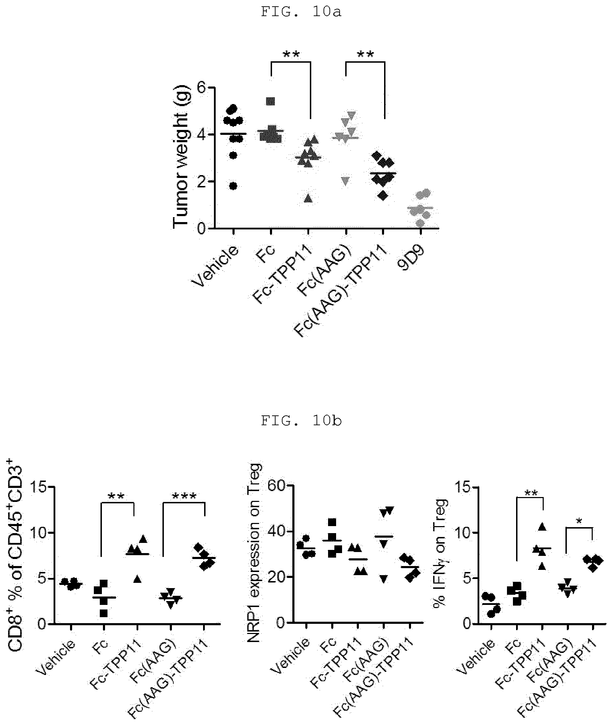

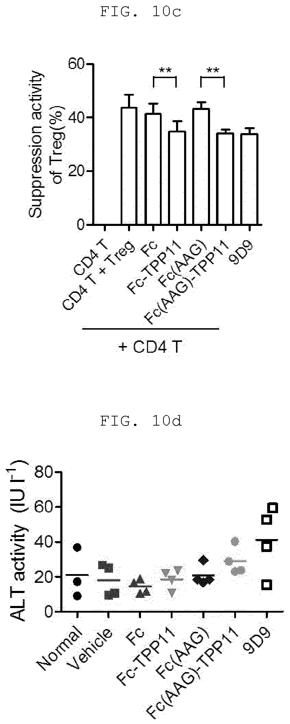

FIG. 10 shows the results of evaluating cell number, cell activity and toxicity using the isolated tumor-infiltrating lymphocytes obtained by sacrificing the mice at 24 hours after the last administration shown in FIG. 9. Specifically, FIG. 10a is a graph showing the results of measuring the weight of the tumors extracted from the sacrifice mice of each administration group; FIG. 10b depicts graphs showing the proportion of cytotoxic T cells (CD45⁺CD3⁺CD8⁺) among the tumor-infiltrating lymphocytes (CD45⁺) isolated from the sacrificed mice of each administration group, the expression level of neuropilin 1 in regulatory T cells (CD45⁺CD4⁺Foxp3⁺), and the amount of interferon-gamma secreted; and FIG. 10c is a graph showing the results of evaluating regulatory T cell inhibitory activity by culturing the regulatory T cells (CD4⁺CD25⁺) isolated from the tumor-infiltrating lymphocytes of each administration group and the CD4 T cells (CD4⁺CD25⁻) isolated from the spleens of non-tumor-grafted mice.

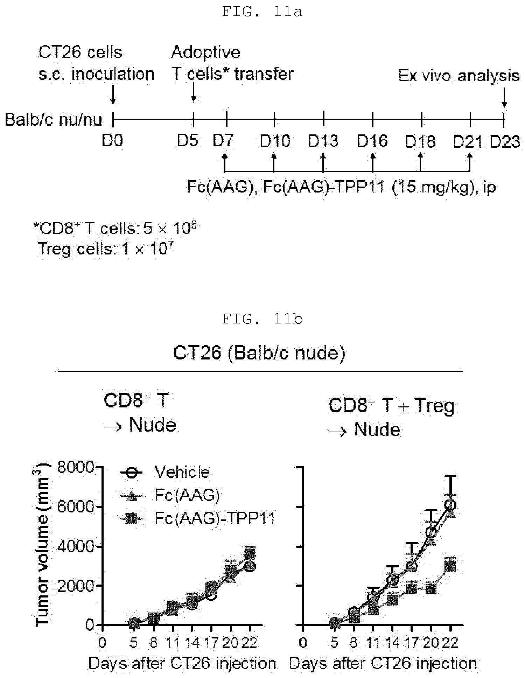

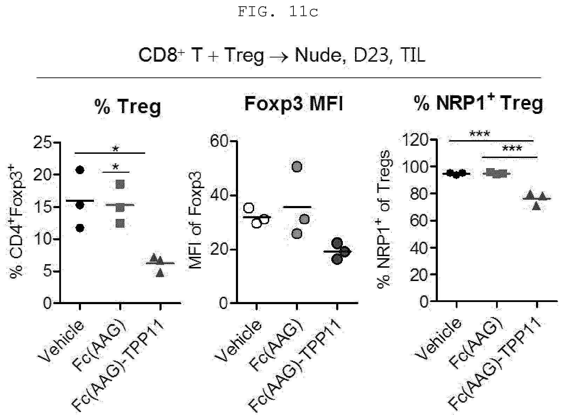

FIG. 11 shows the results of measuring the tumor growth inhibitory activities of Fc(AAG) and Fc(AAG)-TPP11 in Balb/c nude mice adoptively transferred with cytotoxic T cells (CD8⁺) and regulatory T cells (CD4⁺CD25⁺) . Specifically, FIG. 11a is a schedule showing the date of adoptive transfer and the administration interval, dose and administration method of drugs in an experiment on tumor growth inhibitory activity *in vivo* in mice; FIG. 11b depicts graphs showing the changes in tumor volume by administration of Fc(AAG) and Fc(AAG)-TPP11 in the mice; and FIG. 11c depicts graphs showing the proportion of regulatory T cells (CD45⁺CD4⁺Foxp3⁺) among the tumor-infiltrating lymphocytes isolated from the sacrificed mice of each administration group, the expression level of Foxp3, and the proportion of regulatory T cells expressing neuropilin 1.

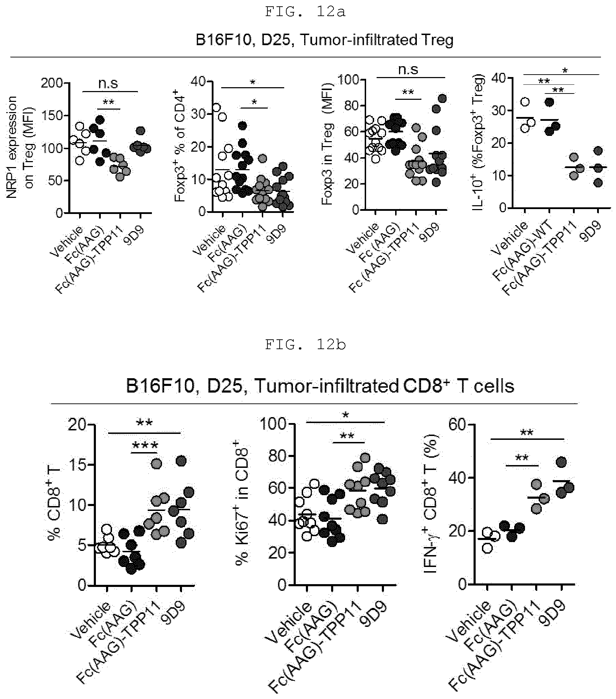

FIG. 12 shows the results of measuring the number and function of B16F10 tumor-infiltrated regulatory T cells (CD4⁺CD25⁺) and cytotoxic T cells (CD45⁺CD3⁺CD8⁺) obtained from the mice sacrificed at 24 hours after the last administration of Fc(AAG)-TPP11 and 9D9 shown in FIG. 9. Specifically, FIG. 12a depicts graphs showing the expression level of neuropilin 1 in tumor-infiltrated regulatory T cells (CD45⁺CD4⁺Foxp3⁺), the proportion of regulatory T cells among CD4⁺ T cells (CD45⁺CD4⁺), the expression level of Foxp3 which is a marker of regulatory T cell inhibitory effect, and the proportion of regulatory T cells secreting interleukin-10, a representative inhibitory cytokine; and FIG. 12b depicts graphs showing the proportion of cytotoxic T cells (CD45⁺CD3⁺CD8⁺) among tumor-infiltrated lymphocytes (CD45⁺), the proliferation ability of the cytotoxic T cells, and the proportion of cytotoxic T cells secreting interferon-gamma.

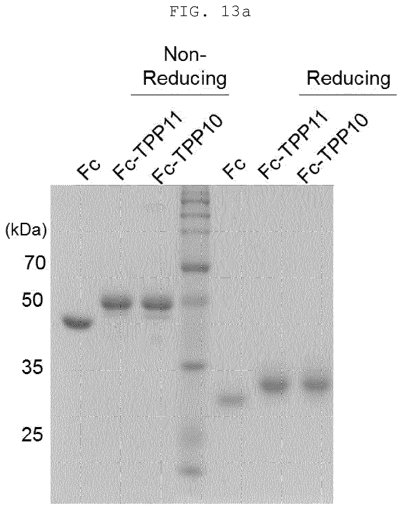

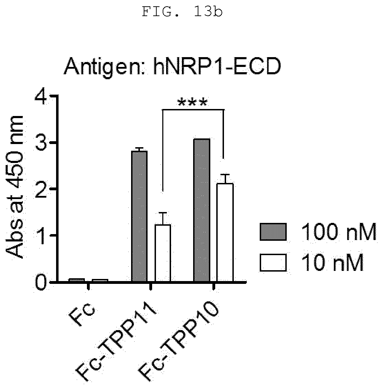

FIG. 13 shows the results of analyzing the expression of Fc-TPP10 fusion protein having increased binding affinity for neuropilin 1 and the binding affinity of the protein for neuropilin 1. Specifically, FIG. 13a shows the results obtained by expressing and purifying Fc-TPP10 fusion protein in animal cells (HEK293F), and then separating 4 µg protein on SDS-PAGE under non-reducing conditions and analyzing the size and combination form of the protein by Coomassie blue staining; and FIG. 13b shows the results of ELISA (enzyme-linked immunosorbent assay) performed to examine whether Fc-TPP10 shows better binding affinity for a human NRP1 extracellular domain (hNRP1-ECD) than Fc-TPP11.

### BEST MODE FOR CARRYING OUT THE INVENTION

Unless defined otherwise, all the technical and scientific terms used herein have the same meaning as those generally understood by one of ordinary skill in the art to which the invention pertains. Generally, the nomenclature used herein and the experiment methods, which will be described below, are those well known and commonly employed in the art.

In one aspect, the present invention is directed to a composition for inhibiting regulatory T cell activity, which contains, as an active ingredient, a peptide that binds specifically to neuropilin 1.

In another aspect, the present invention provides a composition for inhibiting immunosuppression by inhibiting regulatory T cell activity, and a method for inhibiting immunosuppressive response**(another aspect)**. The inhibition of immunosuppression can ultimately enhance immunity, thereby treating either cancer or infectious disease, such as tuberculosis, sepsis or viral hepatitis, which may be caused by immunosuppression.

In still another aspect, the present invention is directed to an immunomodulating agent, an infectious disease therapeutic agent and an anticancer agent, which comprise a composition containing a peptide that binds specifically to neuropilin 1, in which the composition is capable of inhibiting the activity of regulatory T cells that suppress immune response, thereby down-regulating the activity thereof.

In yet another aspect, the present invention is directed to a method for inhibiting the activity of regulatory T cells, which comprises a step of administering a composition containing, as an active ingredient, a peptide that binds specifically to neuropilin 1 to a subject.

In a further aspect, the present invention is directed to an immunomodulating method, which comprises a step of administering a composition which contains, as an active ingredient, a peptide that binds specifically to neuropilin 1 to a subject.

In a still further aspect, the present invention is directed to the use of the composition, for immunomodulation.

In a yet further aspect, the present invention is directed to the use of the composition, for the manufacture of a medicament for immunomodulation.

In another further aspect, the present invention is directed to a method for preventing or treating either cancer or infectious disease such as tuberculosis, sepsis or viral hepatitis, which comprises a step of administering a composition which contains, as an active ingredient, a peptide that binds specifically to neuropilin 1 to a subject.

In another still further aspect, the present invention is directed to the use of the composition, for the prevention or treatment of either cancer or infectious disease such as tuberculosis, sepsis or viral hepatitis.

In another yet further aspect, the present invention is directed to the use of the composition, for the manufacture of a medicament for preventing or treationg either cancer or infectious disease such as tuberculosis, sepsis or viral hepatitis.

In another yet further aspect, the present invention is directed to the use of a peptide binding specifically to neuropilin 1, for the inhibition of regulatory T cell (Treg) activity.

In the present invention, the peptide that binds specifically to neuropilin 1 may comprise one or more sequences selected from the group consisting of TPP11 (SEQ ID NO: 1), TPP1 (SEQ ID NO: 2), TPP8 (SEQ ID NO: 3), and TPP10 (SEQ ID NO: 4), but is not limited thereto:
HTPGNSKPTRTPRR (TPP11, SEQ ID NO: 1),
HTPGNSNQFVLTSTRPPR (TPP1, SEQ ID NO: 2),
HTPGIATRTPR (TPP8, SEQ ID NO: 3), and
HTPGNSKPTRTPR (TPP10, SEQ ID NO: 4).

In the present invention, the peptide that binds specifically to neuropilin 1 may be used alone. Preferably, the peptide may be a fusion protein fused to an antibody (immunoglobulin) heavy-chain constant region (Fc), an antibody or an antibody fragment. When the peptide that binds specifically to neuropilin 1 is provided as a fusion protein fused to an antibody heavy-chain constant region (Fc), an antibody or an antibody fragment, there are advantages in that the peptide exhibits a synergistic effect and has excellent immunomodulatory activity, and thus the therapeutic effect of the peptide on cancer or infectious diseases can be maximized.

The peptide binding specifically to neuropilin 1 may be linked to an Fc, an antibody or fragment thereof by linker-mediated binding, chemical bonding, genetic fusion, or the like.

As used herein, the term "Fc region" or "heavy-chain constant region" means a region comprising an immunoglobulin CH2 domain, a CH3 domain and a hinge domain, which are derived from the antibody. However, for IgE, the term means a region comprising a CH2 domain, a CH3 domain, a CH4 domain and a hinge domain.

In the present invention, the linker is preferably a peptide linker, but is not limited thereto. For example, the peptide linker may comprise a sequence of (GGGGS)n (where n is an integer ranging from 1 to 20), but is not limited thereto.

In the present invention, the peptide may bind to the C-terminus of a heavy-chain constant region (Fc) of the antibody.

In the present invention, the antibody may be selected from the grouop consisting of IgG1, IgG2, IgG3, IgG4, IgM, IgA, IgD and IgE, but is not limited thereto.

In the present invention, the antibody fragment may be Fab, F(ab)₂, scFv, V_{H} or V_{L}, but is not limited thereto.

The antibody fragment means the heavy-chain or light-chain domain of the antibody, or a fragment thereof. For example, the antibody fragment may be Fc, Fab, a heavy-chain constant region fragment (CH1, CH2, or CH3) of an antibody, a heavy-chain variable region fragment (VH), a light-chain constant region fragment (CL), a light-chain variable region fragment (VL), a single-chain variable fragment (scFv), or a fragment thereof.

"Fab" has a structure including variable regions of a light chain and a heavy chain, a constant region of the light chain, and a first constant region (CH1) of the heavy chain with one antigen-binding site. Fab' differs from Fab in that it has a hinge region containing one or more cysteine residues at the C-terminal of the heavy chain CH1 domain. The F(ab')₂ antibody is produced when the cysteine residue of the hinge region of the Fab' forms a disulfide bond.

An "Fv" fragment is an antibody fragment that contains complete antigen recognition and binding sites. Such a region includes a heavy chain variable domain and a light chain variable domain, for example, dimers substantially tightly covalently associated with scFv.

"Single chain Fv" or "scFv" antibody fragment comprises VH and VL domains of the antibody. Such domains are within a single polypeptide chain. The Fv polypeptide may further include a polypeptide linker between the VH domain and the VL domain such that the scFv can form the desired structure for antigen binding.

A single chain Fv (scFv) is connected to a heavy chain variable region and a light chain variable region via a peptide linker by a covalent bond or directly at the C-terminal. Such an antibody fragment can be obtained using a protein hydrolyzing enzyme (for example, when the whole antibody is cleaved with papain, Fab can be obtained, and when whole antibody is cut with pepsin, F(ab')2 fragment can be obtained), and it can also be produced through gene recombinant technology.

The heavy chain constant region can be selected from any one isotype of gamma (γ), mu (µ), alpha (α), delta (δ), and epsilon (ε). Sub-classes have gamma 1 (γ1), gamma 2 (γ2), gamma 3 (γ3), gamma 4 (γ4), alpha 1 (α1), and alpha 2 (α2) types. A constant region of the light chain may have kappa (κ) and lambda (λ) types.

The term "heavy chain" as used herein refers to a full-length heavy chain and fragments thereof including a variable region domain VH including an amino acid sequence with sufficient variable region sequence to confer specificity to an antigen and three constant region domains CH1, CH2, and CH3. The term "light chain" as used herein refers to a full-length heavy chain and fragments thereof including a variable region domain VL including an amino acid sequence with sufficient variable region sequence to confer specificity to an antigen and a constant region domain CL.

Moreover, the antibody fragment may be a monomer, a dimer, or a multimer. A peptide binding specifically to the neuropilin-1 may bind to a heavy chain constant region (Fc) fragment of an antibody, preferably to the N- or C-terminus of Fc.

The "linking" means integrating two molecules having different or same functions or structures, and may be used interchangeably with "fusing". It may be linking or fusing by all physical, chemical or biological methods by which the peptide can be linked.

In the present invention, the antibody or antibody fragment preferably binds specifically to an immune checkpoint-associated antigen, a cancer cell surface-specific antigen, a tumor blood vessel-specific antigen, a tumor microenvironment-specific antigen, a regulatory T cell-specific antigen, or an antigen specific for immune cells that infiltrated into tumor tissue, but is not limited thereto. Specifically, the antigen preferably is an immune checkpoint-associated antigen, but is not limited thereto.

The antigen is preferably selected from the group consisting of EGFR (Epidermal growth factor receptor), ERBB2 (HER2), VEGF (Vascular endothelial growth factor), EpCAM (Epithelial cell adhesion molecule), VEGFR-2 (VEGF receptor-2), c-Met (hepatocyte growth factor receptor), CD20 (cluster of differentiation-20), CD19 (cluster of differentiation-19), CD22 (cluster of differentiation-22), CTLA-4 (cytotoxic T-lymphocyte-associated protein 4), PD-1 (Programmed cell death protein 1), PD-L1 (Programmed death-ligand 1), TIM3 (T-cell immunoglobulin and mucin-domain containing-3), MSLN (Mesothelin), TNFRSF4 (Tumor necrosis factor receptor superfamily member 4, OX40), PSMA (prostate specific membrane antigen), GPC3 (Glypican-3), GD2 ganglioside, CEA (carcinoembryonic antigen), FAP (fibroblast-activation protein), integrin, and fibronectin. More preferably, the antigen is selected from the group consisting of cetuximab, panitumumab, zalutumumab, nimotuzumab, necitumumab, matuzumab, trastuzumab, bevacizumab, ramucirumab, onartuzumab, rituximab, ipilimumab, nivolumab, lambrolizumab, cergutuzumab, and pembrolizumab, but is not limited thereto.

The composition of the present invention may be formulated using a pharmaceutically acceptable carrier and/or an excipient according to a method which can be easily carried out by those having ordinary skill in the art to which the present invention pertains so as to be provided in a unit dosage form or enclosed into a multi-dose container. A pharmaceutical composition according to the present invention may further comprise a pharmaceutically acceptable carrier. The term "pharmaceutically acceptable carrier" refers to a substance which can be added to the active ingredient to help formulate or stabilize the preparation and causes no significant adverse toxicological effects to the patient.

As used herein, the term "pharmaceutically acceptable carrier" refers to a carrier or diluent that does not impair the biological activity and characteristics of a peptide binding specifically to neuropilin 1 contained in the composition according to the present invention and a fusion protein comprising the peptide without irritating an organism. As a pharmaceutically acceptable carrier in a composition that is formulated as a liquid solution, a sterile and biocompatible carrier is used. The pharmaceutically acceptable carrier may be physiological saline, sterile water, Ringer's solution, buffered saline, albumin injection solution, dextrose solution, maltodextrin solution, glycerol, ethanol, or a mixture of two or more thereof. In addition, the composition of the present invention may, if necessary, comprise other conventional additives, including antioxidants, buffers, and bacteriostatic agents. Further, the composition of the present invention may be formulated as injectable forms such as aqueous solutions, suspensions or emulsions with the aid of diluents, dispersants, surfactants, binders and lubricants. In addition, the composition according to the present invention may be formulated in the form of pills, capsules, granules, or tablets. Other carriers are described in, for example, a literature [Remington's Pharmaceutical Sciences (E. W. Martin)].

Pharmaceutically acceptable carriers include sterile aqueous solutions or dispersions and sterile powders for the extemporaneous preparation of sterile injectable solutions or dispersions. The use of such media and agents for pharmaceutically active substances is known in the art. The composition is preferably formulated for parenteral injection. The composition can be formulated as a solid, a solution, a microemulsion, a liposome, or other ordered structures suitable to high drug concentration. The carrier may be a solvent or dispersion medium containing, for example, water, ethanol, polyol (e.g., glycerol, propylene glycol and liquid polyethylene glycol), and suitable mixtures thereof. In some cases, the composition may contain an isotonic agent, for example, sugar, polyalcohol (e.g., mannitol or sorbitol) or sodium chloride. Sterile injectable solutions can be prepared by incorporating the peptide binding specifically to neuropilin 1 or the fusion protein comprising the peptide in the required amount in an appropriate solvent with one or a combination of ingredients enumerated above, as required, followed by filtered sterilization. Generally, dispersions are prepared by incorporating the peptide binding specifically to neuropilin 1 and the fusion protein comprising the peptide into a sterile vehicle, which contains a basic dispersion medium and the required other ingredients from those enumerated above. In the case of sterile powders for the preparation of sterile injectable solutions, the preferred methods of preparation are vacuum drying and freeze-drying techniques, which yield a powder of the active ingredient plus any additional desired ingredient from a previously sterile-filtered solution thereof.

In addition, the composition according to the present invention may be orally or parenterally administered to suffering patients at a dosage and frequency that may vary with the severity of the suffering patients. The compositions may be administered to patients in need as a bolus or by continuous infusion. In another example, the pharmaceutical composition according to the present invention may be administered rectally, intravenously, subcutaneously, intratumorly, intrauterinely, or intracerebrovascularly, but is not limited thereto.

In the present invention, the inhibition of regulatory T cell activity may inhibit immunosuppression caused by regulatory T cells.

In the present invention, the composition may inhibit cancer cell growth by: i) reducing the proportion of regulatory T cells among CD4+ T cells in tumor tissue; or ii) increasing the expression of interleukin-2 and interferon-gamma by inhibiting the activity of regulatory T cells that infiltrated into tumor tissue; or iii) increasing the growth of cytotoxic T cells by inhibiting the activity of regulatory T cells that infiltrated into tumor tissue.

In one example of the present invention, it was confirmed that a fusion protein comprising a peptide binding specifically to neuropilin 1 is constructed, and then the fusion protein inhibits the activity of regulatory T cells.

In another example of the present invention, it was confirmed that as the activity of regulatory T cells as inhibited, the growth of CD4⁺ T cells in colorectal cancer and malignant melanoma tumor *in vivo* increased, the expression of interleukin-2 and interferon-gamma increased, and the growth of cytotoxic T cells increased. Therefore, it was confirmed that the fusion protein of the present invention inhibits the growth of cancer cells by inhibiting the activity of regulatory T cells.

Thus, the composition can be used for the treatment of either cancer or infectious disease, such as tuberculosis, sepsis or viral hepatitis.

In one example, the present invention is directed to a method for treating cancer or various infectious diseases, which may be caused by immunosuppression, which comprises administering a composition comprising a fusion protein containing a peptide binding specifically to neuropilin 1 to a subject in need of treatment, thereby inhibiting the activity of regulatory T cells.

The present invention is also directed to an immunomodulating agent capable of treating various cancers or infectious diseases by use of a composition comprising either the peptide binding specifically to neuropilin 1 or a fusion protein containing the peptide.

Proper administration dose of the composition may be differentially prescribed depending on various factors such as method for formulation, a mode of administration, age, weight, sex, pathological state, diet of patients, administration time, administration route, excretion rate and reaction sensitivity, etc. Preferably, a proper dose of the composition is within the range of 0.001 and 100 mg/kg based on an adult. The term "pharmaceutically effective dose" as used herein refers to an amount sufficient to prevent or treat cancer or infectious disease.

In an aspect, the composition or the treatment method according to the present invention can be applied to cancer. The cancer is cancer that can be treated by inhibiting of regulatory T cell activity, and examples of the cancer include, but are not limited to, colorectal cancer, malignant melanoma, breast cancer, ovarian cancer, head and neck cancer, pancreatic cancer, lung cancer, brain tumor, liver cancer, prostate cancer, bladder cancer, blood cancer (chronic or acute lymphocytic or lymphoblastic leukemia, chronic/acute myelogenous leukemia, etc.), ACTH-producing tumor, carcinoma of the cervix, chronic myelogenous leukemia, T-zone lymphoma, endometriosis, esophageal cancer, gallbladder cancer, Ewing's sarcoma, tongue cancer, Hodgkin's lymphoma, Kaposi's sarcoma, kidney cancer, mesothelioma, multiple myeloma, neuroblastoma, non-Hodgkin Lymphoma, osteosarcoma, ductal carcinoma in situ, penis cancer, retinoblastoma, skin cancer, gastric cancer, thyroid cancer, uterine cancer, testicular cancer, Wilms' tumor, or trophoblastoma. Most preferably, the cancer may be colorectal cancer or malignant melanoma.

In another yet further aspect, the present invention is directed to an anticancer agent comprising the composition. The composition can exhibit direct anticancer effects through a composition comprising, as an active ingredient, either a peptide according to the present invention, which binds specifically to neuropilin 1, or a fusion protein comprising the peptide, or it can be used in combination with other anticancer drugs.

Therefore, the present invention is directed to an anticancer agent comprising a composition comprising, as an active ingredient, either a peptide binding specifically to neuropilin 1 or a fusion protein comprising the peptide.

The composition comprising either the peptide binding specifically to neuropilin 1 or the fusion protein comprising the peptide can be co-administered with other anticancer drugs to inhibit the activity of regulatory T cells, thereby improving the cancer treatment effect. In addition, the composition comprising either the peptide binding specifically to neuropilin 1 or the fusion protein comprising the peptide may further comprise other anticancer drugs.

The composition for co-administration includes a peptide that binds specifically to neuropilin-1 or a fusion protein comprising the peptide, and the components related thereto are the same as the components included in the above-described composition and treatment method. Thus, the description of each constitution applies equally to the method of treating cancer by co-administration.

As used herein, the term "co-administration" means that the fusion protein to which the peptide binding specifically to neuropilin-1 is fused and the anticancer agent may be administered simultaneously, sequentially, or in reverse order, and the fusion protein and the anticancer agent may be administered in a combination of appropriate effective amounts of the active ingredients within the range determined by those skilled in the art. For example, the fusion protein to which the peptide binding specifically to neuropilin-1 is fused and the anticancer agent may be respectively stored in separate containers, and then co-administered simultaneously, sequentially, or in reverse order.

In another aspect, the composition or the treatment method according to the present invention can be applied to infectious disease. The infectious disease includes bacterial and viral infectious diseases, and may preferably be exemplified by tuberculosis, sepsis or viral hepatitis, but is not limited thereto. Specifically, the viral hepatitis disease may be hepatitis A, hepatitis B, or hepatitis C, but is not limited thereto.

### EXAMPLES

Hereinafter, the present invention will be described in further detail with reference to examples. It will be obvious to a person having ordinary skill in the art that these examples are for illustrative purposes only and are not to be construed to limit the scope of the present invention.

### Example 1: Expression and Purification of Fc-TPP11 and 9D9-TPP11

In order to examine whether Fc-TPP11 and 9D9-TPP11 can inhibit the activity of regulatory T cells, Fc-TPP11 and 9D9-TPP11 were expressed and purified.

"Fc-TPP11" in the present invention refers to a fusion protein in which the peptide TPP11 that binds specifically to neuropilin 1 is fused to Fc, and "9D9-TPP11" refers to a fusion protein in which TPP11 is fused to 9D9, an antibody specific for CTLA4.

Specifically, from a vector (KR 10-1551306) for producing a fusion protein (Fc-TPP11) composed of a TPP11 peptide and an antibody heavy-chain constant region (Fc), an antibody heavy-chain constant region CH3 was obtained by treatment with NotI and HindIII restriction enzymes. A DNA of a TPP11-fused DNA in the obtained antibody heavy-chain constant region CH3 was cloning into a vector encoding a wild-type 9D9 heavy chain. The amino acid sequence of the TPP11-fused portion in the antibody heavy-chain constant region CH3 is set forth in SEQ ID NO: 5; the DNA sequence is set forth in SEQ ID NO: 6; and the amino acid sequence of the wild-type 9D9 heavy chain is set forth in SEQ ID NO: 7. In addition, the amino acid sequence encoding the light chain is set forth in SEQ ID NO: 8, and the light chain is set forth in SEQ ID NO: 9. A vector expressing the wild-type 9D9 light chain was used in the same manner.

**[Table 1]**

| Constitution | Sequences | SEQ ID NO: |
|---|---|---|
| 9D9-TPP11 heavy-chain amino acid sequence | METDTLLLWVLLLWVPGSTGDEAKLQESGPVLVKPGASV KMSCKASGYTFTDYYMNWVKQSHGKSLEWIGVINPYNGD TSYNQKFKGKATLTVDKSSSTAYMELNSLTSEDSAVYYC ARYYGSWFAYWGQGTLITVSAKTTAPSVYPLAPVCGDTT GSSVTLGCLVKGYFPEPVTLTWNSGSLSSGVHTFPAVLQ SDLYTLSSSVTVTSSTWPSQSITCNVAHPASSTKVDKKI EPRGPTIKPCPPCKCPAPNLLGGPSVFIFPPKIKDVLMI | SEQ ID NO: 5 |
| | SLSPIVTCVVVDVSEDDPDVQISWFVNNVEVHTAQTQTH REDYNSTLRVVSALPIQHQDWMSGKEFKCKVNNKDLPAP IERTISKPKGSVRAPQVYVLPPPEEEMTKKQVTLTCMVT DFMPEDIYVEWTNNGKTELNYKNTEPVLDSDGSYFMYSK LRVEKKNWVERNSYSCSVVHEGLHNHHTTKSFSRTPGKG GGGSGGGGSGGGGSHTPGNSKPTRTPRR | |
| 9D9-TPP11 heavy-chain DNA sequence | ATGGAGACAGACACACTCCTGCTATGGGTACTGCTGCTC TGGGTTCCAGGTTCCACTGGTGACGAGGCAAAGCTGCAG GAGTCCGGACCCGTGCTGGTGAAGCCTGGAGCCAGCGTG AAGATGTCCTGCAAGGCCTCTGGCTACACCTTCACAGAC TACTATATGAACTGGGTGAAGCAGAGCCACGGCAAGTCC CTGGAGTGGATCGGCGTGATCAACCCCTACAATGGCGAC ACCTCTTATAACCAGAAGTTTAAGGGCAAGGCCACCCTG ACAGTGGATAAGAGCTCCTCTACCGCCTACATGGAGCTG AATAGCCTGACATCCGAGGATTCTGCCGTGTACTATTGT GCCCGGTACTATGGCTCTTGGTTCGCCTATTGGGGCCAG GGCACCCTGATCACAGTGAGCGCCAAAACAACAGCCCCA TCGGTCTATCCACTGGCCCCTGTGTGTGGAGATACAACT GGCTCCTCGGTGACTCTAGGATGCCTGGTCAAGGGTTAT TTCCCTGAGCCAGTGACCTTGACCTGGAACTCTGGATCC CTGTCCAGTGGTGTGCACACCTTCCCAGCTGTCCTGCAG TCTGACCTCTACACCCTCAGCAGCTCAGTGACTGTAACC TCGAGCACCTGGCCCAGCCAGTCCATCACCTGCAATGTG GCCCACCCGGCAAGCAGCACCAAGGTGGACAAGAAAATT GAGCCCAGAGGGCCCACAATCAAGCCCTGTCCTCCATGC AAATGCCCAGCACCTAACCTCTTGGGTGGACCATCCGTC TTCATCTTCCCTCCAAAGATCAAGGATGTACTCATGATC TCCCTGAGCCCCATAGTCACATGTGTGGTGGTGGATGTG AGCGAGGATGACCCAGATGTCCAGATCAGCTGGTTTGTG AACAACGTGGAAGTACACACAGCTCAGACACAAACCCAT AGAGAGGATTACAACAGTACTCTCCGGGTGGTCAGTGCC CTCCCCATCCAGCACCAGGACTGGATGAGTGGCAAGGAG TTCAAATGCAAGGTCAACAACAAAGACCTCCCAGCGCCC ATCGAGAGAACCATCTCAAAACCCAAAGGGTCAGTAAGA GCTCCACAGGTATATGTCTTGCCTCCACCAGAAGAAGAG | SEQ ID NO: 6 |
| | ATGACTAAGAAACAGGTCACTCTGACCTGCATGGTCACA GACTTCATGCCTGAAGACATTTACGTGGAGTGGACCAAC AACGGGAAAACAGAGCTAAACTACAAGAACACTGAACCA GTCCTGGACTCTGATGGTTCTTACTTCATGTACAGCAAG CTGAGAGTGGAAAAGAAGAACTGGGTGGAAAGAAATAGC TACTCCTGTTCAGTGGTCCACGAGGGTCTGCACAATCAC CACACGACTAAGAGCTTCTCCCGGACTCCGGGTAAAGGT GGAGGAGGATCTGGAGGAGGAGGAAGTGGAGGTGGAGGA TCACATACTCCTGGAAATAGCAAACCAACACGCACACCA AGGCGT | |
| 9D9 heavy-chain amino acid sequence | METDTLLLWVLLLWVPGSTGDEAKLQESGPVLVKPGASV KMSCKASGYTFTDYYMNWVKQSHGKSLEWIGVINPYNGD TSYNQKFKGKATLTVDKSSSTAYMELNSLTSEDSAVYYC ARYYGSWFAYWGQGTLITVSAKTTAPSVYPLAPVCGDTT GSSVTLGCLVKGYFPEPVTLTWNSGSLSSGVHTFPAVLQ SDLYTLSSSVTVTSSTWPSQSITCNVAHPASSTKVDKKI EPRGPTIKPCPPCKCPAPNLLGGPSVFIFPPKIKDVLMI SLSPIVTCVVVDVSEDDPDVQISWFVNNVEVHTAQTQTH REDYNSTLRVVSALPIQHQDWMSGKEFKCKVNNKDLPAP IERTISKPKGSVRAPQVYVLPPPEEEMTKKQVTLTCMVT DFMPEDIYVEWTNNGKTELNYKNTEPVLDSDGSYFMYSK LRVEKKNWVERNSYSCSVVHEGLHNHHTTKSFSRTPGK | SEQ ID NO: 7 |
| 9D9 light-chain amino acid sequence | METDTLLLWVLLLWVPGSTGDDIVMTQTTLSLPVSLGDQ ASISCRSSQSIVHSNGNTYLEWYLQKPGQSPKLLIYKVS NRFSGVPDRFSGSGSGTDFTLKISRVEAEDLGVYYCFQG SHVPYTFGGGTKLEIKRADAAPTVSIFPPSSEQLTSGGA SVVCFLNNFYPKDINVKWKIDGSERQNGVLNSWTDQDSK DSTYSMSRTLTLTKDEYERHNSYTCEATHKTSTSPIVKS FNRNEC | SEQ ID NO: 8 |
| 9D9 light-chain DNA sequence | ATGGAGACAGACACACTCCTGCTATGGGTACTGCTGCTC TGGGTTCCAGGTTCCACTGGTGACGACATCGTGATGACC CAGACCACACTGTCCCTGCCCGTGTCTCTGGGCGATCAG GCCTCTATCAGCTGCCGGAGCTCCCAGAGCATCGTGCAC TCCAACGGCAATACATACCTGGAGTGGTATCTGCAGAAG CCCGGCCAGTCCCCTAAGCTGCTGATCTACAAGGTGTCT | SEQ ID NO: 9 |
| | AACCGGTTCAGCGGCGTGCCAGACAGATTTTCCGGCTCT GGCAGCGGCACCGATTTCACACTGAAGATCTCCAGGGTG GAGGCAGAGGACCTGGGCGTGTACTATTGTTTCCAGGGC TCTCACGTGCCCTATACCTTTGGCGGCGGCACAAAGCTG GAGATCAAGCGCGCTGATGCTGCACCAACTGTATCCATC TTCCCACCATCCAGTGAGCAGTTAACATCTGGAGGTGCC TCAGTCGTGTGCTTCTTGAACAACTTCTACCCCAAAGAC ATCAATGTCAAGTGGAAGATTGATGGCAGTGAACGACAA AATGGCGTCCTGAACAGTTGGACTGATCAGGACAGCAAA GACAGCACCTACAGCATGAGCAGAACCCTCACGTTGACC AAGGACGAGTATGAACGACATAACAGCTATACCTGTGAG GCCACTCACAAGACATCAACTTCACCCATTGTCAAGAGC TTCAACAGGAATGAGTGT | |

FIGS. 2b and 2c show schematic views of the animal cell expression vectors in the 9D9-TPP11 expression system.

The light-chain and heavy-chain expression vectors were transiently transfected and protein was expressed and purified. In a shaking flask, HEK293-F cells suspension-growing in serum-free FreeStyle 293 expression medium (Invitrogen) were transfected with a mixture of a plasmid and polyethylenimine (PEI) (Polyscience). For 200 mL transfection in a shaking flask (Corning), HEK293-F cells were seeded into 100 ml of medium at a density of 2 x 10⁶ cells/ml and cultured at 130 rpm under 8% CO₂. Heavy-chain and light-chain plasmids suitable for producing each monoclonal antibody were diluted in 10 ml of FreeStyle 293 expression medium (Invitrogen) to 125 µg heavy chain and 125 µg light chain (a total of 250 µg (2.5 µg/ml)), and the dilution was mixed with 10 ml of medium containing 750 µg (7.5 µg/ml) of PEI and was incubated at room temperature for 10 minutes. Next, the incubated mixed medium was added to 100 ml of the above-seeded cells and incubated for 4 hours at 150 rpm under 8% CO₂, and then the remaining 100 ml of FreeStyle 293 expression medium was added thereto, followed by incubation for 7 days. With reference to a standard protocol, protein was purified from the collected cell culture supernatant. Antibody was applied to a Protein A Sepharose column (GE healthcare), followed by washing with PBS (pH 7.4). The antibody was eluted with 0.1 M glycine buffer at pH 3.0, and then the sample was immediately neutralized with 1M Tris buffer (pH 9.0). The eluted antibody fraction was concentrated by dialysis while replacing buffer with PBS (pH 7.4). The purified protein was measured for absorbance at a wavelength of 562 nm by use of the solution in a BCA protein assay kit (Thermo) and quantified according to the plotted standard curve. The yield of the obtained 9D9-TPP11 antibody did not significantly differ from that of the wild type 9D9 antibody, indicating that the antibody was well purified.

### Example 2: Evaluation of the Binding Affinities of 9D9-TPP11 for Neuropilin 1 and CTLA-4

The binding affinities of 9D9-TPP11, expressed and purified in Example 1, for neuropilin 1 and CTLA-4, were analyzed comparatively with those of Fc-TPP11 and the wild-type antibody 9D9.

FIG. 3a shows the results of ELISA (enzyme-linked immunosorbent assay) performed to examine whether constructed 9D9-TPP11 shows binding affinity for a neuropilin 1-b1b2 domain (human NRP1-b1b2, hNRP1-b1b2), like Fc-TPP11, in comparison with 9D9

Specifically, a 96-well plate (SPL, Korea) was coated with a human neuropilin 1-b1b2 domain (0.1 µg /well) at 25°C for 1 hour, and then washed with TBS (pH 7.4) and blocked with 4% BSA-containing TBS (pH 7.4) at 25°C for 1 hour. Next, **the plate** was incubated with various concentrations (10, 1 and 0.1 nM) of each of Fc-TPP11, 9D9 and 9D9-TPP11 at 25°C for 1 hour, followed by washing three times with TBS. The plate was incubated with an HRP-conjugated anti-human antibody heavy-chain domain antibody (horseradish peroxidase anti-human Fc mAb, Thermo, USA) at 25°C for 1 hour, followed by washing three times with TBS. Thereafter, 3,3',5,5'-tetramethylbenzidine (TMB, Sigma) substrate was added, and the absorbance at 450 nm was measured using a microplate reader (Biotek Cytation 3, Korea).

As a result, it was confirmed that in comparison with the non-TPP11-fused wild-type antibody 9D9, the binding affinity of 9D9-TPP11 for the human neuropilin 1-b1b2 domain was similar to the binding affinity of Fc-TPP11 for the human neuropilin 1-b1b2 domain.

FIG. 3b shows the results of ELISA performed to examine whether constructed 9D9-TPP11 shows binding affinity for mouse CTLA-4 antigen and human CTLA-4 antigen in the same manner as the wild-type antibody 9D9 that shows binding affinity for CTLA-4.

Specifically, a 96-well plate (SPL, Korea) was coated with a human neuropilin CTLA-4 (hCTLA-4) and a mouse CTLA-4 (mCTLA-4) (0.5 µg /well) at 25°C for 1 hour, and then washed with TBS (pH 7.4) and blocked with 4% BSA-containing TBS (pH 7.4) at 25°C for 1 hour. Next, the plate was incubated with various concentrations (10, 1 and 0.1 nM) of each of 9D9 and 9D9-TPP11 at 25°C for 1 hour, followed by washing three times with TBS. As the isotype of the 9D9 antibody is a mouse IgG2a, the plate was incubated with an HRP-conjugated anti-mouse antibody heavy-chain domain antibody (horseradish peroxidase anti-mouse Fc mAb, Santa Cruz) at 25°C for 1 hour, followed by washing three times with TBS. Thereafter, 3,3',5,5'-tetramethylbenzidine (TMB, Sigma) substrate was added, and the absorbance at 450 nm was measured using a microplate reader (Biotek Cytation 3, Korea).

As a result, it was confirmed that in comparison with the non-TPP11-fused wild-type antibody, the binding ability of 9D9-TPP11 for mouse CTLA-4 antigen was similar to that of 9D9, and it was confirmed that, like 9D9 having no cross-reactivity to human CTLA-4 antigen, both 9D9 and 9D9-TPP11 showed no binding affinity for human CTLA-4 antigen.

### Example 3: Evaluation of the Abilities of Fc-TPP11 and 9D9-TPP11 to Inhibit Regulatory T Cell Activity

Since the binding affinities of the Fc-TPP11 and 9D9-TPP11, expressed in Example 1, for neuropilin 1 and CTLA-4 antigen, were confirmed in Example 2, whether they would inhibit regulatory T cell activity by binding to the neuropilin 1 and CTLA-4 expressed in the regulatory T cells was evaluated.

FIG. 4 shows the results of CD4 T cell growth assay and cytokine ELISA assay performed to measure the effects of Fc-TPP11 and 9D9-TPP11 against the activity of regulatory T cells that inhibit the growth of CD4⁺ T cells (CD4⁺).

Specifically, spleen was dissected from normal BALB/c mice, and then the spleen was crushed in a 60 mm dish containing a 70-micron mesh and medium. To the cells obtained by centrifugation at 1500 rpm at 4°C for 5 minutes, red blood cell lysis buffer was added to lyse red blood cells. Then, the cells were washed with PBS and incubated with FITC-conjugated anti-CD4 antibody (e-Bioscience) and PE-fused anti-CD25 antibody (e-Bioscience) at 4°C for 30 minutes. After washing with PBS, CD4 T cells (CD4⁺CD25⁻) and regulatory T cells (CD4⁺CD25⁺) were isolated by FACS Aria III (BD biosciences, Korea). Thereafter, a 96-well plate coated with anti-CD3 antibody (1 µg/ml) and anti-CD28 antibody (0.5 µg/ml) at 4°C for 20 hours was treated with the isolated CD4 T cells (2 x 10⁵ cells/well) and regulatory T cells (4 x 10⁴ cells/well) together with each of Fc, Fc-TPP11, 9D9 and 9D9-TPP11 (0.5 µM). After incubation at 37°C for 2 days, 50 µL of the supernatant was recovered from each well and subjected to cytokine (IL-2, IFNγ) ELISA. 50 µL of medium was added to each well which was then further incubated for 2 days, and then CellTiter-Glo Luminescent assay (CTG assay, Promega) was performed. This assay measures the growth rate of CD4 T cells based on the principle according to which luminescence is generated in proportion to the amount of ATP when a substrate is added. 80 µL of a CTG reaction solution was added to each well and incubated on a shaker at 25°C for 10 minutes, and then the cell lysate was transferred to a white 96-well plate, and the luminescence signal was measured using a microplate reader (Biotek Cytation 3, Korea). At this time, a higher luminescence signal value indicates that more growth of the CD4 T cells occurred.

As a result, it was confirmed that when the regulatory T cells were co-cultured with the CD4 T cells, the growth of the CD4 T cells was inhibited by the regulatory T cells, and thus the luminescence signal value decreased compared to the luminescence signal value of the CD4 T cells alone, which were not co-cultured with the regulatory T cells and thus grew more. In addition, it was likewise confirmed that when the CD4 T cells and the regulatory T cells were treated with each of Fc-TPP11, 9D9 and 9D9-TPP11 while they were cultured, the activity of the regulatory T cells decreased and the signal of CD4 T cell growth increased (FIG. 4a).

Next, the concentrations of interleukin-2 and interferon-gamma in the cell culture medium incubated at 37°C for 2 days under the same conditions as described above were measured. A 96-well plate was coated with a human interleukin-2 or human interferon-gamma capture antibody (e-bioscience) for 12 hours, and then washed PBST (PBS with 0.1% Tween-20) and blocked with 1% BSA (PBS with 1% bovine serum albumin) at room temperature for 1 hour. After washing with PBST (PBS with 0.1% Tween-20), the recovered supernatant was diluted five-fold with 1% BSA, and 100 µl of the dilution was added to each well, and then incubated at room temperature for 2 hours. After washing with PBST (PBS with 0.1% Tween-20), each well was incubated with biotin-conjugated anti-interleukin-2 and anti-interferon-gamma detection antibodies (e-bioscience) at room temperature for 1 hour. After washing with PBST (PBS with 0.1% Tween-20), each well was incubated with avidin-conjugated horseradish peroxidase (HRP) (e-bioscience) at room temperature for 30 minutes, and then washed with PBST (PBS with 0.1% Tween-20). Next, a 3,3',5,5"-tetramethylbenzidine (TMB, sigma-aldrich) substrate was added and the absorbance at 450 nm was measured using a microplate reader (Biotek Cytation 3, Korea).

As a result, as shown in FIGS. 4b and 4c, it was confirmed that the secretion of the cytokines interleukin-2 and interferon-gamma from the CD4 T cells during cell growth also increased when treated with each of Fc-TPP11, 9D9 and 9D9-TPP11. At this time, the regulatory T cell activity inhibitory ability of 9D9-TPP11 capable of targeting both CTLA-4 and neuropilin 1 significantly increased compared to that of 9D9 that targets CTLA-4 in the regulatory T cells, indicating that the interferon-gamma secretion ability of the CD4 T cells significantly increased. This suggests that 9D9-TPP11 targeted both neuropilin 1 and CTLA-4, which are different antigens expressed in the regulatory T cells, thereby inhibiting both the growth and activity of the regulatory T cells.

### Example 4: Evaluation of the Abilities of Fc-TPP11 and 9D9-TPP11 to Inhibit Colorectal Cancer Growth in vivo

Since it was confirmed in Example 3 that the fusion peptides Fc-TPP11 and 9D9-TPP11 comprising the neuropilin 1-specific peptide TPP11 had the ability to inhibit the activity of regulatory T cells *in vitro,* whether the same effect of Fc-TPP11 and 9D9-TPP11 would also appear *in vivo* in mice was examined.

FIG. 5 shows the results of measuring the tumor growth inhibitory activities of Fc-TPP11 and 9D9-TPP11 *in vivo* in BALB/c mice.

Specifically, CT26 cells (1 x 10⁶ cells/mouse) were diluted in 150 µL of PBS and grafted subcutaneously into five-week-old female BALB/c mice (Charles River Japan). Mice with tumors having similar sizes (average volume: 120 to 150 mm³) were randomly grouped into treatment groups, and as shown in FIG. 5a, each of Fc and Fc-TPP11 was injected intraperitoneally at a dose of 10 mg/kg once every two days (a total of seven times), and each of 9D9 and 9D9-TPP11 was injected intraperitoneally at a dose of 5 mg/kg once every three days (a total of four times). The tumor was measured twice a week, and the tumor volume (V) was calculated using the following equation: V = length x width² / 2.

As a result, as shown in FIG. 5b, it was confirmed that Fc-TPP11, 9D9 and 9D9-TPP11 exhibited tumor growth inhibitory ability, compared to the control vehicle and Fc. In addition, as shown in FIG. 5c, it was confirmed that administration of Fc-TPP11, 9D9 or 9D9-TPP11 showed little or no change in body weight, compared to administration of the control, and thus had no toxicity.

FIG. 5d is a graph showing the change in tumor volume of each mouse of each administration group as a function of days in the results shown in FIG. 5b.

### Example 5: Evaluation of the Abilities of Fc-TPP11 and 9D9-TPP11 to Inhibit Regulatory T Cell Activity in vivo

In order to examine whether the tumor growth inhibitory abilities of Fc-TPP11 and 9D9-TPP11, confirmed in Example 4, would be due to the inhibition of regulatory T cell activity, tumor was extracted from the mice after the last administration and analyzed.

FIG. 6 shows the results obtained by sacrificing mice at 24 hours after the last administration shown in Example 4, separating lymphocytes and tumor-infiltrating lymphocytes from the spleens, and evaluating cell activity *in vitro* using the isolated cells.

### 5-1: FACS Analysis of Lymphocytes and Tumor-Infiltrating Lymphocytes

FIG. 6a shows the results of flow cytometry (FACS) performed to analyze the proportion of cytotoxic T cells (CD8⁺) among the tumor-infiltrating lymphocytes obtained by sacrificing the mice of each administration group.

Specifically, at 24 hours after the last administration in Example 4, the mice of each administration group were sacrificed, the tumor was extracted therefrom, and then crushed in a 60-mm plate containing a 70-micron mesh and PBS. To the cells obtained by centrifugation, red blood cell lysis buffer was added to lyse red blood cells, followed by washing with PBS. To stain cytotoxic T cells among the cells obtained by crushing the tumor, the cells were incubated with an FITC-conjugated antibody (e-Bioscience) recognizing the lymphocyte marker CD45 and an eFlour660-conjugated antibody recognizing CD8a, at 4°C for 30 minutes. After washing with PBS, cytotoxic T cells (CD45⁺CD8⁺) were analyzed by a flow cytometer (FACS Calibur) (BD biosciences, Korea).

As a result, it could be seen that the proportion of tumor-infiltrated cytotoxic T cells in the Fc-TPP11-administered group increased compared to that in the groups administered with each of control vehicle and Fc, and that the proportion of tumor-infiltrated cytotoxic T cells in the group administered with 9D9-TPP11 capable of targeting both CTLA-4 and neuropilin 1 increased compared to that in the group administered with 9D9 capable of targeting CTLA-4 in regulatory T cells (FIG. 6a).

### 5-2: Effect of Regulatory T Cells on CD4⁺ T Cells

FIG. 6b shows the results of evaluating the inhibitory activity of regulatory T cells against the growth of CD4⁺ T cells by co-culturing the regulatory T cells (CD4⁺CD25⁺) isolated from the tumor-infiltrating lymphocytes of each administration group and the CD4⁺ T cells (CD4⁺CD25⁻) isolated from the spleen of non-tumor-grafted mice, and measuring the concentration of interferon-gamma secreted by CD4⁺ T cells.

Specifically, in the same manner as described in Example 5-1 above, mice were sacrificed, and then spleen and tumor were extracted therefrom and crushed to obtain cells. From the obtained cells, regulatory T cells were isolated using a mouse regulatory T cell (CD4⁺CD25⁺) isolation kit (Miltenyi Biotec). First, the cells were incubated with a cocktail of biotin-conjugated anti-mouse CD8a, CD11b, CD45R, CD49b and Ter119 antibodies, antibody-magnetic beads recognizing biotin, and a PE-conjugated antibody recognizing CD25, at 4°C for 15 minutes. The above-treated cells were loaded onto an LD column, and only the cells that did flow out of the bottom of the column were collected. At this time, the collected cells were CD4 T cells (CD4⁺CD25⁻) and regulatory T cells (CD4⁺CD25⁺), which were not targeted by the cocktail of anti-mouse CD8a, CD11b, CD45R, CD49b and Ter119 antibodies. The collected CD4 T cells and regulatory T cells were centrifuged, and then incubated with antibody-magnetic beads recognizing PE at 4°C for 15 minutes. When the above-treated cells are loaded onto an MS column, the cells that did flow out of the bottom of the column without binding to the column can be regarded as CD4 T cells (CD4⁺CD25⁻), and the cells that did bind to the column can be regarded as regulatory T cells (CD4⁺CD25⁺). According to this method, CD4 T cells (CD4⁺CD25⁻) were obtained from the spleen cells of non-tumor-grafted normal mice, and regulatory T cells (CD4⁺CD25⁺) were obtained from the cells obtained from the tumor of each administration group. Thereafter, in a 96-well round bottom plate (SPL, Korea) coated with anti-CD3 antibody (1 µg/ml) and anti-CD28 antibody (0.5 µg/ml) at 4°C for 20 hours, the CD4 T cells (1x10⁵ cells/well) and regulatory T cells (2 x 10⁴ cells/well) obtained by isolation as described above were cultured. After culture at 37°C for 2 days, 50 µL of the supernatant was collected from each well, and ELISA was performed to compare the secretion of interferon-gamma. 50 µL of medium was added to each well which was then further incubated for 2 days, and then CellTiter-Glo Luminescent assay (CTG assay, Promega) was performed. 100 µL of a CTG reaction solution was added to each well which was then incubated on a shaker at 25°C for 15 minutes, and then the cell lysate was transferred to a white 96-well plate, and the luminescence signal was measured using a microplate reader (Biotek Cytation 3, Korea). At this time, a higher luminescence signal value indicates that more growth of the CD4⁺ T cells occurred.

As a result, it was confirmed that the CD4⁺ T cell growth inhibitory activity of regulatory T cells in the Fc-TPP11-administered group decreased compared to that in the control Fc-administered group, and that the CD4⁺ T cell growth inhibitory activity of regulatory T cells in the group administered with 9D9-TPP11 capable of targeting both CTLA-4 and neuropilin 1 significantly decreased compared to that in the group administered with 9D9 that targets CTLA-4 in regulatory T cells.

Next, for the supernatant collected after 2 days of co-culture, the amount of interferon-gamma secreted by CD4⁺ T cells was measured by ELISA.

Specifically, a 96-well plate for ELISA was coated with mouse interferon-gamma capture antibody (e-Bioscience) for 12 hours, and then washed with PBST (PBS with 0.1% Tween-20) and blocked with 1% BSA (PBS with 1% bovine serum albumin) at 25°C for 1 hour. After washing with PBST, the collected culture medium was diluted five-fold with 1% BSA, and 100 µl of the dilution was added to each well which was then incubated at 25°C for 2 hours. After washing with PBST, each well was incubated with biotin-conjugated interferon-gamma detection antibody (e-Bioscience) at 25°C for 1 hour. After washing with PBST, each well was incubated with avidin-conjugated horseradish peroxidase (HRP) (e-Bioscience) at 25°C for 30 minutes, and then washed with PBST. Next, a 3,3',5,5''-tetramethylbenzidine (TMB, sigma-aldrich) substrate, and the absorbance at 450 nm was measured using a microplate reader (Biotek Cytation 3, Korea).

As a result, as shown in FIG. 6c, it was confirmed that the secretion of the cytokine interferon-gamma from the CD4 T cells during cell growth increased when treated with each of Fc-TPP11, 9D9 and 9D9-TPP11. At this time, the regulatory T cell activity inhibitory ability of 9D9-TPP11 capable of targeting both CTLA-4 and neuropilin 1 significantly increased compared to that of 9D9 that targets CTLA-4 in the regulatory T cells, indicating that the secretion of interferon-gamma from the CD4⁺ T cells significantly increased. This suggests that 9D9-TPP11 targeted both neuropilin 1 and CTLA-4, which are different antigens expressed in the regulatory T cells, thereby inhibiting both the growth and activity of the regulatory T cells.

### Example 6: Construction, Expression and Purification of Fc(AAG) and Fc(AAG)-TPP11

In Example 5, the mouse tumor growth inhibitory activities of the fusion peptides Fc-TPP11 and 9D9-TPP11 comprising the neuropilin 1-specific peptide TPP11 were confirmed. At this time, in order to confirm only the effect of regulatory T cells, except for tumor inhibitory activity caused by antibody-dependent cell cytotoxicity (ADCC), among tumor growth inhibitory activities, an Fc mutant was constructed by substituting the residue lysine at positions 234 and 235 with alanine and substituting at proline at position 329 with glycine so that an Fc gamma receptor would not bind to the Fc region. The constructed Fc mutant was named Fc(AAG). TPP11 was fused to the Fc(AAG), thereby constructing Fc(AAG)-TPP11. The tumor growth inhibitory activity of the constructed Fc(AAG)-TPP11 by regulatory T cell targeting was analyzed in comparison with Fc-TPP11 capable of inducing antibody-dependent cytotoxicity.

Thus, Fc(AAG) and Fc(AAG)-TPP11 were first constructed.

Specifically, DNAs coding for Fc and Fc-TPP11 were cloned in-frame into animal cell expression vectors to have hinge-CH2-CH3 using NotI/HindII restriction enzymes and synthetic oligonucleotides (Macrogen, Korea) by a site-directed mutagenesis method which is performed by those skilled in the art. Table 2 below shows the amino acid and DNA sequences of Fc(AAG)-TPP11 in which TPP11 is fused to Fc (AAG) .

**[Table 2]**

| Constitution | Sequences | SEQ ID NO: |
|---|---|---|
| Fc(AAG)-TPP11 amino acid sequence | TCPPCPAPEAAGGPSVFLFPPKPKDTLMISRTPEVTCVV VDVSHEDPEVKFNWYVDGVEVHNAKTKPREEQYNSTYRV VSVLTVLHQDWLNGKEYKCKVSNKALGAPIEKTISKAKG QPREPQVYTLPPSRDELTKNQVSLTCLVKGFYPSDIAVE WESNGQPENNYKTTPPVLDSDGSFFLYSKLTVDKSRWQQ GNVFSCSVMHEALHNHYTQKSLSLSPGKGGGGSGGGGSG GGGSHTPGNSKPTRTPRR | SEQ ID NO: 10 |
| Fc(AAG)-TPP11 DNA sequence | ACATGCCCACCGTGCCCAGCACCTGAAGCCGCCGGGGGA CCGTCAGTCTTCCTCTTCCCCCCAAAACCCAAGGACACC CTCATGATCTCCCGGACCCCTGAGGTCACATGCGTGGTG | SEQ ID NO: 11 |
| | GTGGACGTGAGCCACGAAGACCCTGAGGTCAAGTTCAAC TGGTACGTGGACGGCGTGGAGGTGCATAATGCCAAGACA AAGCCGCGGGAGGAGCAGTACAACAGCACGTACCGTGTG GTCAGCGTCCTCACCGTCCTGCACCAGGACTGGCTGAAT GGCAAGGAGTACAAGTGCAAGGTCTCCAACAAAGCCCTC GGCGCCCCCATCGAGAAAACCATCTCCAAAGCCAAAGGG CAGCCTCGAGAACCACAGGTGTACACCCTGCCCCCATCC CGGGATGAGCTGACCAAGAACCAGGTCAGCCTGACCTGC CTGGTCAAAGGCTTCTATCCCAGCGACATCGCCGTGGAG TGGGAGAGCAATGGGCAGCCGGAGAACAACTACAAGACC ACGCCTCCCGTGCTGGACTCCGACGGCTCCTTCTTCCTC TACAGCAAGCTCACCGTGGACAAGAGCAGGTGGCAGCAG GGGAACGTCTTCTCATGCTCCGTGATGCATGAGGCTCTG CACAACCACTACACGCAGAAGAGCCTCTCCCTGTCGCCG GGTAAAGGTGGAGGAGGATCTGGAGGAGGAGGAAGTGGA GGTGGAGGATCACATACTCCTGGAAATAGCAAACCAACA CGCACACCAAGGCGT | |
| Fc(AAG) amino acid sequence | TCPPCPAPEAAGGPSVFLFPPKPKDTLMISRTPEVTCVV VDVSHEDPEVKFNWYVDGVEVHNAKTKPREEQYNSTYRV VSVLTVLHQDWLNGKEYKCKVSNKALGAPIEKTISKAKG QPREPQVYTLPPSRDELTKNQVSLTCLVKGFYPSDIAVE WESNGQPENNYKTTPPVLDSDGSFFLYSKLTVDKSRWQQ GNVFSCSVMHEALHNHYTQKSLSLSPGK | SEQ ID NO: 12 |
| Fc-TPP11 amino acid sequence | TCPPCPAPELLGGPSVFLFPPKPKDTLMISRTPEVTCVV VDVSHEDPEVKFNWYVDGVEVHNAKTKPREEQYNSTYRV VSVLTVLHQDWLNGKEYKCKVSNKALPAPIEKTISKAKG QPREPQVYTLPPSRDELTKNQVSLTCLVKGFYPSDIAVE WESNGQPENNYKTTPPVLDSDGSFFLYSKLTVDKSRWQQ GNVFSCSVMHEALHNHYTQKSLSLSPGKGGGGSGGGGSG GGGSHTPGNSKPTRTPRR | SEQ ID NO: 13 |
| Fc amino acid sequence | TCPPCPAPELLGGPSVFLFPPKPKDTLMISRTPEVTCVV VDVSHEDPEVKFNWYVDGVEVHNAKTKPREEQYNSTYRV VSVLTVLHQDWLNGKEYKCKVSNKALPAPIEKTISKAKG QPREPQVYTLPPSRDELTKNQVSLTCLVKGFYPSDIAVE WESNGQPENNYKTTPPVLDSDGSFFLYSKLTVDKSRWQQ | SEQ ID NO: 14 |
| | GNVFSCSVMHEALHNHYTQKSLSLSPGK | |

The Fc(AAG) and Fc(AAG)-TPP11 expression vectors were transiently transfected and protein was expressed and purified. In a shaking flask, HEK293-F cells suspension-growing in serum-free FreeStyle 293 expression medium (Invitrogen) were transfected with a mixture of a plasmid and polyethylenimine (PEI)(Polyscience). For 30 mL transfection in a shaking flask (Corning), HEK293-F cells were seeded into 25 ml of medium at a density of 2 x 10⁶ cells/ml and cultured at 130 rpm under 8% CO₂. Fc(AAG) and Fc(AAG)-TPP11 expression plasmids suitable for producing each protein were diluted in 3 ml of FreeStyle 293 expression medium (Invitrogen) to 37.5 µg, and the dilution was mixed with 5 ml of medium containing 112.5 µg of PEI and was incubated at room temperature for 10 minutes. Next, the incubated mixed medium was added to 25 ml of the above-seeded cells and incubated for 7 days at 150 rpm under 8% CO₂. With reference to a standard protocol, protein was purified from the collected cell culture supernatant. Antibody was applied to a Protein A Sepharose column (GE healthcare), followed by washing with PBS (pH 7.4). The antibody was eluted with 0.1 M glycine buffer at pH 3.0, and then the sample was immediately neutralized with 1M Tris buffer (pH 9.0). The eluted antibody fraction was concentrated by dialysis while replacing buffer with PBS (pH 7.4). The purified protein was measured for absorbance at a wavelength of 562 nm by use of the solution in a BCA protein assay kit (Thermo) and quantified according to the plotted standard curve. The yield of the obtained Fc(AAG) and Fc(AAG)-TPP11 proteins did not significantly differ from that of the Fc(AAG) and Fc(AAG)-TPP11 proteins before introduction of a mutant, indicating that the proteins were well purified.

### Example 7: Evaluation of the Abilities of Fc-TPP11 and Fc(AAG)-TPP11 to Inhibit Colorectal Cancer Growth in Vivo

In order to compare the tumor growth inhibitory ability by regulatory T cell targeting of Fc(AAG) and Fc (AAG) -TPP11, constructed, expressed and purified in Example 6, with that of Fc-TPP11 capable of inducing antibody-dependent cytotoxicity, the tumor inhibitory activities of Fc, Fc-TPP11, Fc (AAG), Fc(AAG)-TPP11 and 9D9 *in vivo* in BALB/c mice were measured.

FIG. 7 shows the results of measuring the tumor growth inhibitory activities of Fc, Fc-TPP11, Fc(AAG)-TPP11 and 9D9 *in vivo* in BALB/c mice.

Specifically, CT26 cells (1 x 10⁶ cells/mouse) were diluted in 150 µL of PBS and grafted subcutaneously into five-week-old female BALB/c mice (Charles River Japan). Mice with tumors having similar sizes (average volume: 120 to 150 mm³) were randomly grouped into treatment groups, and as shown in FIG. 7a, each of Fc, Fc-TPP11, Fc (AAG) and Fc(AAG)-TPP11 was injected intraperitoneally at a dose of 10 mg/kg once every two days (a total of seven times), and 9D9 was retro-orbital injected at a dose of 2.5 mg/kg once every three days (a total of three times). The tumor was measured twice a week, and the tumor volume (V) was calculated using the following equation: V = length x width² / 2.

As a result, as shown in FIG. 7b, it was confirmed that the groups administered with each of Fc-TPP11, Fc(AAG)-TPP11 and 9D9 exhibited tumor growth inhibitory ability compared to the groups administered with each of the control vehicle, Fc and Fc(AAG). In addition, the tumor growth inhibitory ability did not significantly differ between Fc-TPP11 and Fc(AAG)-TPP11. That is, it could be seen that the inhibition of tumor growth by the mechanism of antibody-dependent cytotoxicity caused by binding to the Fc gamma receptor did not appear, the inhibition of tumor growth appeared through TPP11, a peptide that binds specifically to neuropilin 1.

In addition, as shown in FIG. 7c, it could be confirmed that there was little or no change in the body weight of the mice administered with each of Fc-TPP11, Fc(AAG)-TPP11 and 9D9, compared to the groups administered with each of the control vehicle, Fc and Fc(AAG).

FIG. 7d is a graph showing the change in tumor volume of each mouse of each administration group as a function of days in the results shown in FIG. 7b. It was confirmed that the mice of each administration group showed similar tumor growth rates without significant difference.

### Example 8: Evaluation of Mechanism by In Vivo Tumor-Infiltrating Lymphocyte Analysis of Groups Administered with Fc-TPP11 and Fc(AAG)-TPP11

In order to examine whether the *in vivo* tumor inhibition by administration of Fc-TPP11 and Fc(AAG)-TPP11 in Example 7 is an effect obtained by targeting neuropilin 1 of regulatory T cells through TPP11, the mice of each of the groups administered with Fc(AAG) and Fc(AAG)-TPP11 in Example 7 were sacrificed at 24 hours after the fifth administration, and tumor-infiltrated lymphocytes were isolated therefrom, and the proportions of cytotoxic T cells and regulatory T cells among the tumor-infiltrated lymphocytes were analyzed by flow cytometry (FACS).

Specifically, the extracted tumor was cut to a weight of 0.5 to 1.0 g and crushed in the same manner as that in Example 5 to obtain cells. To stain cytotoxic T cells, the obtained cells were incubated with an APC-conjugated antibody (e-Bioscience) recognizing the lymphocyte marker CD45, a PE-cy5-conjugated antibody (e-Bioscience) recognizing CD3, and a PE-cy5-conjugated antibody (e-Bioscience) recognizing CD8, at 4°C for 30 minutes. In addition, to stain regulatory T cells, the obtained cells were incubated with an FITC-conjugated antibody (e-Bioscience) recognizing CD45 and a PE-conjugated antibody (e-Bioscience) recognizing CD4, at 4°C for 30 minutes (e-Bioscience). After washing with PBS, Foxp3 staining was also performed using a Foxp3 staining kit in the same manner as described in Example 5. After washing with PBS, cytotoxic T cells (CD45⁺CD3⁺CD8⁺) and regulatory T cells (CD45⁺CD4⁺Foxp3⁺) were analyzed by a flow cytometer (FACS Aria III, BD bioscience, Korea).

As a result, it was observed that the proportion of cytotoxic T cells among the tumor-infiltrated lymphocytes increased in the group administered with Fc(AAG)-TPP11 compared to the groups with each of control vehicle and Fc(AAG). However, it was confirmed that the proportion of regulatory T cells among CD4⁺ T cells (CD45⁺CD4⁺) in the tumor decreased in the group administered with Fc(AAG)-TPP11 compared to the groups administered with each of control vehicle and Fc(AAG) (FIG. 8a).

In addition, in order to examine whether the increase in the proportion of cytotoxic T cells in the group administered with Fc(AAG)-TPP11 is an effect obtained because Fc(AAG)-TPP11 inhibited the activity of regulatory T cells, the inhibitory activity of regulatory T cells was evaluated by co-culturing the regulatory T cells (CD4⁺CD25⁺) isolated from the tumor-infiltrated lymphocytes of each administration group and the CD4 T cells (CD4⁺CD25⁻) isolated from the spleen of non-tumor-grafted mice.

Specifically, in the same manner as described in Example 5-2, regulatory T cells (2 x 10⁴ cells/well) isolated from the tumor-infiltrated lymphocytes of the groups administered with vehicle, Fc(AAG) and Fc(AAG)-TPP11, and CD4 T cells (1 x 10⁵cells/well) obtained by crushing the spleen of non-tumor-grafted normal mice were co-cultured in a 96-well round bottom plate pre-coated with anti-CD3 and CD28 antibodies, at 37°C under 5% CO₂ for 3 days. After 3 days, 100 µl of a CTG substrate was added to each well which was then incubated at 25°C for 15 minutes, thereby lysing the cells. The cell lysate was transferred to a white 96-well plate, and luminescence was measured using a microplate reader.

As a result, it was confirmed that the CD4⁺ T cell growth inhibitory ability of the regulatory T cells obtained from the group administered with Fc(AAG)-TPP11 decreased compared to that in the control group Fc(AAG)-administered group, like the results obtained for the groups administered with each of Fc and Fc-TPP11 (FIG. 8b) . Thus, it was confirmed that the mechanism by which Fc(AAG)-TPP11 exhibits CT26 tumor growth inhibitory activity in the Balb/c mouse model is the mechanism by which NRP1-targeting Fc(AAG)-TPP11 reduces the proportion of regulatory T cells among CD4+ T cells in tumor tissue and inhibits the activity of regulatory T cells that infiltrated into tumor tissue, thereby increasing the growth of cytotoxic T cells in tumor tissue.

### Example 9: Evaluation of the Abilities of Fc-TPP11 and Fc(AAG)-TPP11 to Inhibit Growth of Malignant Melanoma In Vivo

In Example 8, it was confirmed that Fc-TPP11 and Fc(AAG)-TPP11 exhibited the activity of inhibiting colorectal cancer growth in BALB/c mice. Whether this tumor growth inhibitory ability would also appear in other tumor models was additionally examined through a malignant melanoma model.

FIG. 9 shows the results of measuring the B16F10 tumor (malignant melanoma) growth inhibitory activities of Fc-TPP11, Fc(AAG)-TPP11 and 9D9 *in vivo* in C57BL/6 mice.

Specifically, B16F10 cells (1 x 10⁶ cells/mouse) were diluted in 150 µL of PBS and grafted subcutaneously into five-week-old female C57BL/6 mice (NaraBio, Korea). Mice with tumors having similar sizes (average volume: 120 to 150 mm³) were randomly grouped into treatment groups, and as shown in FIG. 9a, each of Fc, Fc-TPP11, Fc(AAG), and Fc(AAG)-TPP11 were injected intraperitoneally at a dose of 10 mg/kg once every two days (a total of seven times), and 9D9 was injected intraperitoneally at a dose of 2.5 mg/kg once every three days (a total of five times). The tumor was measured twice a week, and the tumor volume (V) was calculated using the following equation: V = length x width² / 2.

As a result, as shown in FIG. 9b, it was confirmed that tumor growth was inhibited in the groups administered with each of Fc-TPP11 and Fc(AAG)-TPP11 compared to the groups the groups administered with each of control vehicle, Fc and Fc(AAG), and that CTLA-4-targeting 9D9 also exhibited tumor growth inhibitory ability. In addition, in FIG. 9c, it was confirmed that there was little or no change in the body weight of the mice administered with each of Fc-TPP11, Fc(AAG)-TPP11 and 9D9, compared to the control group, indicating that Fc-TPP11, Fc(AAG)-TPP11 and 9D9 had no toxicity.

FIG. 9d is a graph showing the change in tumor volume of each mouse of each administration group as a function of days in the results shown in FIG. 9b. It was confirmed that the mice of each administration group showed similar tumor growth rates without significant difference.

### Example 10: Evaluation of Mechanism and Drug Toxicity by Analysis of In Vivo Malignant Melanoma-Infiltrated Lymphocytes of Groups Administered with Fc-TPP11 and Fc(AAG)-TPP11

Similarly to Example 8 in which the *in vivo* tumor inhibitory mechanism was evaluated by administration of Fc-TPP11 and Fc(AAG)-TPP11, the mechanism of *in vivo* tumor inhibition in groups administered with Fc-TPP11 and Fc(AAG)-TPP11 was evaluated using a malignant melanoma model.

First, FIG. 10a shows the results of the weight of each tumor extracted from each mouse sacrificed at 24 hours after the last administration in Example 9.

As a result, it was confirmed that the tumor weight in the groups administered with each of Fc-TPP11, Fc(AAG)-TPP11 and 9D9 decreased compared to that in the groups administered with each of control vehicle, Fc and Fc(AAG). These results are similar to the results obtained for the tumor growth inhibitory abilities of Fc-TPP11, Fc(AAG)-TPP11 and 9D9 shown in the tumor growth curve obtained by measuring the tumor volume at various time points as shown in FIG. 9b.

### 10-1: Analysis of In Vivo Malignant Melanoma-Infiltrated Lymphocytes

Next, in order to investigate the mechanism of Fc-TPP11 and Fc(AAG)-TPP11, tumor-infiltrated lymphocytes were isolated from the mice sacrificed at 24 hours after the last administration of Fc(AAG) and Fc(AAG)-TPP11 in Example 9, and the proportion of cytotoxic T cells in the tumor, the expression of regulatory T cells on the surface of regulatory T cells, and the amount of interferon-gamma secreted from regulatory T cells were examined.

Specifically, the extracted tumor was cut to a weight of 0.5 to 1.0 g and crushed in the same manner as described in Example 5 to obtain cells. To stain cytotoxic T cells, the obtained cells were incubated with an APC-conjugated antibody (e-Bioscience) recognizing the lymphocyte marker CD45, a PE-cy5-conjugated antibody (e-Bioscience) recognizing CD3, and a PE-cy5-conjugated antibody (e-Bioscience) recognizing CD8, at 4°C for 30 minutes. In addition, to stain regulatory T cells, the obtained cells were incubated with an FITC-conjugated antibody (e-Bioscience) recognizing CD45, a PE-conjugated antibody (e-Bioscience) recognizing CD4, and an APC-conjugated antibody (e-Bioscience) recognizing neuropilin 1 at 4°C for 30 minutes (e-Bioscience). After washing with PBS, Foxp3 staining was also performed using a Foxp3 staining kit in the same manner as described in Example 5. After washing with PBS, the proportion of cytotoxic T cells (CD45⁺CD3⁺CD8⁺) were analyzed by a flow cytometer (FACS Calibur, BD bioscience, Korea).

As a result, it was confirmed that the proportion of cytotoxic T cells among the tumor-infiltrated lymphocytes increased in the groups administered with each of Fc-TPP11 and Fc(AAG)-TPP11 compared to the groups administered with each of control vehicle, Fc and Fc(AAG). In addition, when the expression level of neuropilin 1 on the surface of regulatory T cells was compared between the groups, it was observed that the expression level of neuropilin 1 tended to decrease in the groups administered with each of the fusion proteins Fc-TPP11 and Fc(AAG)-TPP11 comprising TPP11 compared to the control group (FIG. 10b).

In addition, according to the recent literature, it was reported that in the case of regulatory T cells lacking neuropilin 1, the expression of interferon-gamma and interferon-gamma receptor in the regulatory T cells increases, and thus the cytotoxic T cell growth inhibitory activity of the regulatory T cells decreases. Thus, in order to examine whether the mechanism by which the secretion of interferon-gamma from regulatory T cells is increased when Fc-TPP11 and Fc(AAG)-TPP11 capable of targeting neuropilin 1 are administered, tumor-infiltrated lymphocytes were isolated from each administration group after the last administration in Example 9, the expression level of interferon-gamma in the regulatory T cells obtained from each administration group was analyzed by intracellular staining and flow cytometry.

Specifically, in the same manner as described in Example 8, tumor was extracted, and then crushed to obtain a cell suspension in which tumor cells and tumor-infiltrated lymphocytes were present as a mixture. The cell suspension was prepared at a density of 2 x 10⁶ cells per administration group and incubated under stimulation with anti-CD3 antibody (1 µg/ml) and anti-CD28 antibody (0.5 µg/ml) at 37°C for 12 hours. Thereafter, the cells were treated with the protein transport inhibitor Golgi plug (BD bioscience) for 6 hours according to the manufacturer's protocol. After 6 hours, the cells were collected and incubated with an FITC-conjugated antibody recognizing CD4 and a PE-conjugated antibody recognizing CD25, at 4°C for 30 minutes. To stain cytokines in the cells, the cells were incubated with 100 µl of a BD Cytofix/Cytoperm solution (BD bioscience) at 4°C for 20 minutes, so that the cells were fixed and permeabilized. In a fixation and permeabilization solution, the cells were incubated with a PE-cy5-conjugated antibody at 4°C for 30 minutes. After washing with a fixation and permeabilization solution, the cells were analyzed by a flow cytometer (FACS Calibur), and the expression level of stained interferon-gamma in regulatory T cells (CD4⁺CD25⁺) among the tumor-infiltrated lymphocytes obtained from the tumor of each administration group was measured.

As a result, it was confirmed that the expression level of interferon-gamma significantly increased in the groups administered with each of Fc-TPP11 and Fc(AAG)-TPP11 compared to the groups administered with each of control vehicle, Fc and Fc(AAG) (FIG. 10b). This is the same result as the phenomenon occurring in neuropilin 1-lacking regulatory T cells reported in the existing literature, and it could be confirmed that the neuropilin 1-targeting peptide TPP11 increases the expression of interferon-gamma in regulatory T cells, thereby reducing the cytotoxic T cell growth inhibitory activity of the regulatory T cells.

### 10-2: Evaluation of Regulatory T Cell Activity

Additionally, FIG. 10c shows the results of evaluating the regulatory T cell inhibitory effect by culturing the regulatory T cells (CD4⁺CD25⁺) isolated from the tumor-infiltrated lymphocytes of each administration group in Example 9 and the CD4 T cells isolated from the spleen of non-tumor-grafted mice.

Specifically, in the same manner as described in Example 5-2, the regulatory T cells isolated from the tumor-infiltrated lymphocytes of the groups administered with Fc, Fc-TPP11, Fc(AAG) and Fc(AAG)-TPP11 and the CD4 T cells obtained by crushing the spleen of non-tumor-grafted normal mice were cultured in a 96-well round bottom plate under stimulation with anti-CD3 and CD28 at 37°C under 5% CO₂ for 3 days. After 3 days, 100 µ1 of a CTG substrate was added to each well which was then incubated at 25°C for 15 minutes, thereby lysing the cells. The cell lysate was transferred to a 96-well white plate, and luminescence was measured using a microplate reader.

As a result, it was confirmed that even in the malignant melanoma model, the activity of regulatory T cells decreased in the groups administered with each of Fc-TPP11 and Fc(AAG)-TPP11 compared to the groups administered with each of control Fc and Fc (AAG) (FIG. 10c).

Next, in order to examine whether or not Fc-TPP11 and Fc(AAG)-TPP11 show toxicity by inhibiting the activity of regulatory T cells (immunocytes) in mice, hepatotoxicity levels were compared in addition to changes in the body weight of mice.

Specifically, mice were sacrificed at 24 hours after the last administration in Example 9, and about 300 to 500 µl of blood was collected each of four mice per administration group and centrifuged at 12,000 rpm and 25°C for 30 minutes to separate serum. To measure hepatotoxicity, ALT (Alanine Transaminase Activity) assay was performed. In this assay, because the enzyme ALT present in the liver enters blood as the liver is damaged, whether drug administration causes hepatotoxicity can be determined by separating serum from collected blood and measuring the concentration of the ALT enzyme in the isolated serum. The isolated serum was diluted five-fold, and the assay was performed using an ALT assay kit (AsanPharm, Korea) according to the manufacturer's protocol.

As a result, the ALT level in the 9D9-administered group tended to increase compared to that in other administration groups, but this increase was not significant, and considering that the normal ALT level is less than 35 U/ml, the administration group showing hepatotoxicity compared to the normal mice and the control Fc and Fc(AAG) groups was not observed (FIG. 10d). That is, it was confirmed that Fc-TPP11 and Fc(AAG)-TPP11 did not show toxicity resulting the inhibition of regulatory T cells (immunocytes) in the mice.

### Example 11: Evaluation of the Ability of Fc (AAG)-TPP11 to Inhibit T Cell-Specific Colorectal Cancer Growth In Vivo

In order to examine whether the *in vivo* tumor growth inhibitory effect of Fc(AAG)-TPP11 administration in Examples 7 to 10 is an effect dependent on regulatory T cells (CD4⁺CD25⁺), the tumor growth inhibitory activity of Fc(AAG)-TPP11 adoptively transferred with cytotoxic T cells (CD8⁺) and regulatory T cells (CD4⁺CD25⁺) were measured.

FIG. 11 shows the results of measuring the tumor growth inhibitory activities of Fc (AAG) and Fc(AAG)-TPP11 in Balb/c nude mice adoptively transferred with cytotoxic T cells (CD8⁺) and regulatory T cells (CD4⁺CD25⁺).

Specifically, CT26 cells (1 x 10⁶ cells/mouse) were diluted in 150 µL of PBS and grafted subcutaneously into 5-week-old female BALB/c mice (Charles River Japan), and on 15 days after the grafting, the cytotoxic T cells (CD8⁺) and regulatory T cells (CD4⁺CD25⁺) to be adoptively transferred were isolated from mouse spleen and inguinal lymph nodes having an average volume of 400 to 500 mm³. The spleen and inguinal lymph nodes were crushed using a mesh in a Petri dish, and then washed with 10 ml of medium containing 2% FBS. Next, to the lymph nodes, 1 ml of red blood cell lysis buffer was added to lyse red blood cells, followed by washing with PBS, thereby preparing a cell suspension. The lymphocytes were stained with a PE-conjugated antibody recognizing CD8 at 4°C for 30 minutes, and washed with cold PBS (pH 7.4), and then anti-PE microbeads (Miltenyi Biotec) were bound thereto for 15 minutes, and cytotoxic T cells (CD8⁺) were isolated from the lymphocytes using a MACS separator and an LS column (Miltenyi Biotec). To separate regulatory T cells (CD4⁺CD25⁺), a regulatory T cell isolation kit (Miltenyi Biotec) was used. First, the CD4⁺ T cells were enriched using biotinylated antibody, and the lymphocytes were stained with a PE-conjugated antibody recognizing CD25 at 4°C for 30 minutes, washed with cold PBS (pH 7.4), and then bound with anti-PE microbeads (Miltenyi Biotec) for 15 minutes, and CD4⁺CD25⁺T cells were isolated therefrom using a MACS separator and an LS column (Miltenyi Biotec). The cytotoxic T cells (CD8⁺) and the regulatory T cells (CD4⁺CD25⁺) showed a purity of 95% or higher.

In order to confirm whether the *in vivo* tumor growth inhibitory effect of Fc(AAG)-TPP11 administration is dependent on regulatory T cells (CD4⁺CD25⁺), CT26 cells (1 x 10⁶ cells/mouse) were diluted in 150 µL of PBS and grafted subcutaneously into 5-week-old female BALB/c nude mice (Charles River Japan), and then the mice having similar tumor sizes (average volume: 120 to 150 mm³) were randomly grouped into treatment groups. As shown in FIG. 11a, on day 5, cytotoxic T cells (CD8⁺, 5 x 10⁶ cells/mouse) and regulatory T cells (CD4⁺CD25⁺, 5 x 10⁶ cells/mouse) were adoptively transferred by intravenous injection, and each of Fc(AAG) and Fc(AAG)-TPP11 was injected intraperitoneally at a dose of 15 mg/kg once every three days (a total of six times). The tumor was measured every three days, and the tumor volume (V) was calculated using the following equation: V=length x width² / 2.

As a result, as shown in FIG. 11b, it was confirmed that Fc(AAG)-TPP11 exhibited no tumor growth inhibitory effect in the mice adoptively mice with the cytotoxic T cells (CD8⁺) alone, but exhibited a tumor growth inhibitory effect in the mice adoptively mice with both the cytotoxic T cells (CD8⁺) and the regulatory T cells (CD4⁺CD25⁺).

In addition, in order to examine whether the tumor growth inhibitory ability in the Fc(AAG)-TPP11-administered group is an effect obtained by targeting neuropilin 1 of regulatory T cells (CD4⁺CD25⁺) through TPP11 as observed in Examples 8 and 9, the mice of the groups administered with Fc(AAG) and Fc(AAG)-TPP11 in Example 11 were sacrificed at 48 hours after the six administration, and tumor-infiltrated lymphocytes were isolated therefrom, and the proportion of regulatory T cells among the tumor-infiltrated lymphocytes and the expression level of neuropilin 1 were analyzed by flow cytometry (FACS).

Specifically, the extracted tumor was cut to a weight of 0.5 to 1.0 g and crushed in the same manner as described in Example 5 to obtain cells. To stain cytotoxic T cells, the obtained cells were incubated with a FITC-conjugated antibody (e-Bioscience), a PE-conjugated antibody (e-Bioscience) recognizing CD4, and an APC-conjugated antibody (e-Bioscience) recognizing neuropilin 1, at 4°C for 30 minutes. After washing with PBS, Foxp3 staining was also performed using a Foxp3 staining kit in the same manner as described in Example 5. After washing with PBS, regulatory T cells (CD45⁺CD4⁺Foxp3⁺) were analyzed by a flow cytometer (FACS Aria III, BD bioscience, Korea).

As a result, it was confirmed that the proportion of the regulatory T cells (CD45⁺CD4⁺Foxp3⁺) and the expression level of the regulatory T cell inhibitory effect marker Foxp3 decreased in the Fc(AAG)-TPP11-administered group compared to the group administered with each of control vehicle and Fc(AAG) (FIG. 11c). In addition, the expression level of neuropilin 1 on the surface of the regulatory T cells decreased in the group administered with the fusion protein Fc(AAG)-TPP11 comprising TPP11, compared to the control group (FIG. 11c). Thus, it was confirmed that Fc(AAG)-TPP11 reduces the proportion of regulatory T cells among CD4⁺ T cells in tumor tissue by targeting NRP1, and inhibits the expression of Foxp3 important for the activity of regulatory T cells that infiltrated into tumor tissue, thereby exhibiting CT26 tumor growth inhibitory ability only in the presence of regulatory T cells.

### Example 12: Evaluation of Whether Fc (AAG)-TPP11 Reduces the Activity of Tumor-Infiltrated Regulatory T Cells and Increase the Function of Cytotoxic T Cells

Since it was confirmed in Example 11 that the *in vivo* tumor growth inhibitory effect of Fc(AAG)-TPP11 administration is dependent on regulatory T cells (CD4⁺CD25⁺), whether the mechanism of the tumor formation inhibitory effect of Fc(AAG)-TPP11 is due to the decrease in number and function of regulatory T cells by a decrease in the expression level of neuropilin 1 in tumor-infiltrated regulatory T cells (CD4⁺CD25⁺) was examined. In addition, whether the decrease in the number and function of regulatory T cells leads to an increase in the number and function of tumor-infiltrated cytotoxic T cells (CD8⁺) was also analyzed.

Specifically, the extracted tumor was cut to a weight of 0.5 to 1.0 g and crushed in the same manner as described in Example 5 to obtain cells. To stain cytotoxic T cells, the obtained cells were incubated with a FITC-conjugated antibody (e-Bioscience), a PE-conjugated antibody (e-Bioscience) recognizing CD4, and an APC-conjugated antibody (e-Bioscience) recognizing neuropilin 1, at 4°C for 30 minutes. After washing with PBS, Foxp3 staining was also performed using a Foxp3 staining kit in the same manner as described in Example 5. To stain cytotoxic T cells, cells were incubated with an APC-conjugated antibody (e-Bioscience) recognizing the lymphocyte marker CD45, a PE-cy5-conjugated antibody (e-Bioscience) recognizing CD3, and a PE-conjugated antibody (e-Bioscience) recognizing CD8, at 4°C for 30 minutes. In addition, for staining with ki-67 capable of evaluating the proliferation ability of cytotoxic T cells, the cells were fixed and permeabilized using a Foxp3/Transcription Factor Staining Buffer Set (Thermo Fisher Scientific), a reagent which is used to stain intracellular protein. Thereafter, the cells were stained with an FITC-conjugated antibody (e-Bioscience) recognizing ki-67 at 4°C for 30 minutes, and permeabilization buffer was added thereto, and the cells were analyzed by a flow cytometer (FACS Aria III, BD bioscience, Korea).

Regulatory T cells inhibit the function of cytotoxic T cells by the inhibitory cytokine interleukin-10. On the other hand, it is known that cytotoxic T cells inhibit cancer cell growth by secreting cytokines such as interferon-gamma. In order to examine whether Fc(AAG)-TPP11 would reduce the interleukin-10 secretory function of regulatory T cells and increase the interferon-gamma secretory function of cytotoxic T cells, tumor-infiltrated lymphocytes were isolated using Ficoll.

Specifically, a 15 ml test tube was filled with 5 ml of Ficoll (GE Healthcare). A tumor lysate was mixed with 5 ml of RPMI 1640 medium, and the mixture was added to the Ficoll-containing test tube so as not to mix with Ficoll, and was centrifuged in a "no break" state at 750 g for 20 minutes. Next, the buffy coat formed on the Ficoll was recovered and washed twice with PBS (pH 7.4), and then tumor-infiltrated lymphocytes were isolated. To evaluate the cytokine secretory abilities of regulatory T cells and cytotoxic cells in the isolated tumor-infiltrated lymphocytes, the cell suspension was prepared at a density of 1x10⁶ per administration group and incubated with phorbol 12-myristate 13-acetate (PMA; 100 ng/mL) under stimulation with ionomycin (500 ng/mL) at 37°C for 8 hours. The cells were treated with the protein transport inhibitor Golgi plug (BD bioscience) according to the manufacturer's protocol for 6 hours before the cells were collected. To analyze the interleukin-10 secretory ability of regulatory T cells, the cells were collected and incubated with an FITC-conjugated antibody recognizing CD4 and a PE-conjugated antibody recognizing CD25, at 4°C for 30 minutes. To stain cytokines in the cells, the cells were incubated with 100 µl of a BD Cytofix/Cytoperm solution (BD bioscience) at 4°C for 20 minutes, so that the cells were fixed and permeabilized. In a fixation and permeabilization solution, the cells were incubated with a PE-cy5-conjugated antibody at 4°C for 30 minutes. After washing with a fixation and permeabilization solution, the cells were analyzed by a flow cytometer (FACS Calibur), and the proportion of interleukin-10-secreting regulatory T cells among the regulatory T cells (CD4⁺CD25⁺) obtained from the tumor of each administration group was measured. To analyze the interferon-gamma secretory ability of cytotoxic T cells, the cells were collected and incubated with an FITC-conjugated antibody recognizing CD8 and a PE-conjugated antibody recognizing CD3, at 4°C for 30 minutes. To stain cytokines in the cells, the cells were incubated with 100 µl of a BD Cytofix/Cytoperm solution (BD bioscience) at 4°C for 20 minutes, so that the cells were fixed and permeabilized. In a fixation and permeabilization solution, the cells were incubated with a PE-cy5-conjugated antibody at 4°C for 30 minutes. After washing with a fixation and permeabilization solution, the cells were analyzed by a flow cytometer (FACS Calibur), and the proportion of interferon-gamma-secreting cytotoxic T cells among the cytotoxic T cells (CD3⁺CD8⁺) obtained from the tumor of each administration group was measured.

As a result, the expression of neuropilin 1 in regulatory T cells (CD45⁺CD4⁺Foxp3⁺) decreased in the Fc(AAG)-TPP11-administered group compared to the groups administered with each of control vehicle and Fc(AAG), and the proportion of regulatory T cells (CD45⁺CD4⁺Foxp3⁺) among CD4+ T cells (CD45⁺CD4⁺) decreased (FIG. 12a). In particular, in the group administered with Fc(AAG)-TPP11, the expression level of Foxp3, a marker of regulatory T cell inhibitory effect, decreased, and the proportion of regulatory T cells secreting the inhibitory cytokine interleukin-10 also greatly decreased (FIG. 12a). Thus, it was confirmed that Fc(AAG)-TPP11 reduces the expression level of neuropilin 1 in regulatory T cells, thereby reducing the number and function of the regulatory T cells.

In addition, it was confirmed that the proportion of cytotoxic T cells (CD3⁺CD8⁺) among tumor-infiltrated lymphocytes was higher in the Fc(AAG)-TPP11-administered group than in the groups administered with each of control vehicle and Fc(AAG), and not only the proliferation ability but also interferon-gamma secretory ability of these cytotoxic T cells were higher than those in the control group (FIG. 12b). Thus, it was confirmed that Fc(AAG)-TPP11 reduces the number and function of tumor-infiltrated regulatory T cells, and as a result, increases the number and function of cytotoxic T cells, thereby suppressing tumor formation.

### Example 13: Construction of Fc-TPP10 Having Increased Binding Affinity for Neuropilin 1 and Evaluation of Binding Affinity for Neuropilin 1

### 13-1: Construction, Expression and Purification of Fc-TPP10

Based on Fc-TPP11 used in Examples 1 to 10, Fc-TPP10 was constructed, which has increased binding affinity for neuropilin 1, and thus can have enhanced effects such as inhibition of regulatory T cell activity. Through analysis of the conventional peptide TPP11 (SEQ ID NO: 1) that binds specifically to neuropilin 1, a peptide TPP10 sequence (SEQ ID NO: 4) expected to have increased binding affinity for neuropilin 1 was designed and synthesized, and then Fc-TPP10 was constructed, expressed and purified.
HTPGNSKPTRTPR (TPP10, SEQ ID NO: 4)

"Fc-TPP10" in the present invention refers to a fusion protein in which the peptide TPP10 that binds specifically to neuropilin 1 is fused to an antibody heavy-chain constant region (Fc).

Specifically, in the same manner as construction of the vector for producing Fc-TPP11 in Example 1, a DNA for producing the fusion protein Fc-TPP10 comprising the TPP10 peptide was cloned.

FIG. 13a shows the results obtained by expressing and purifying Fc-TPP10 fusion protein in animal cells (HEK293F), and then separating 4 µg protein on SDS-PAGE under nonreducing conditions and analyzing the size and combination form of the protein by Coomassie blue staining.

The Fc, Fc-TPP11 and Fc-TPP10 expression vectors were transiently transfected and protein was expressed and purified. In a shaking flask, HEK293-F cells suspension-growing in serum-free FreeStyle 293 expression medium (Invitrogen) were transfected with a mixture of a plasmid and polyethylenimine (PEI)(Polyscience). For 30 mL transfection in a shaking flask (Corning), HEK293-F cells were seeded into 25 ml of medium at a density of 2 x 10⁶ cells/ml and cultured at 130 rpm under 8% CO₂. Fc, Fc-TPP11 and Fc-TPP10 expression plasmids suitable for producing each protein were diluted in 3 ml of FreeStyle 293 expression medium (Invitrogen) to 37.5 µg, and the dilution was mixed with 5 ml of medium containing 112.5 µg of PEI and was incubated at room temperature for 10 minutes. Next, the incubated mixed medium was added to 25 ml of the above-seeded cells and incubated for 7 days at 150 rpm under 8% CO₂. With reference to a standard protocol, protein was purified from the collected cell culture supernatant. Antibody was applied to a Protein A Sepharose column (GE healthcare), followed by washing with PBS (pH 7.4). The antibody was eluted with 0.1 M glycine buffer at pH 3.0, and then the sample was immediately neutralized with 1M Tris buffer (pH 9.0). The eluted antibody fraction was concentrated by dialysis while replacing buffer with PBS (pH 7.4). The purified protein was measured for absorbance at a wavelength of 562 nm by use of the solution in a BCA protein assay kit (Thermo) and quantified according to the plotted standard curve. The yield of the obtained Fc-TPP10 protein did not significantly differ from that of the Fc and Fc-TPP11 proteins, indicating that the proteins were well purified.

### 13-2: Evaluation of the Binding Affinity of Fc-TPP10 Fusion Protein for Neuropilin 1

In order to examine whether the Fc-TPP10 fusion protein comprising the TPP10 peptide designed through sequencing of the TPP11 peptide would show better binding affinity for a human NRP1 extracellular domain (hNRP1-ECD) than Fc-TPP11, ELISA (Enzyme linked immunosorbent assay) was performed.

Specifically, a 96-well plate (SPL, Korea) was coated with a human neuropilin 1 extracellular domain protein (0.2 µg /well) at 4°C for 20 hour, and then washed with TBS (pH 7.4) and blocked with 3% BSA-containing TBS (pH 7.4) at 25°C for 3 hour. Next, the plate was incubated with various concentrations (100 and 10 nM) of each of Fc, Fc-TPP11 and Fc-TPP10 at 25°C for 1 hour, followed by washing three times with TBS. The plate was incubated with an HRP-conjugated anti-human antibody heavy-chain domain antibody (horseradish peroxidase anti-human Fc mAb, Thermo, USA) at 25°C for 1 hour, followed by washing three times with TBS. Thereafter, 3,3',5,5'-tetramethylbenzidine (TMB, Sigma) substrate was added, and the absorbance at 450 nm was measured using a microplate reader (Biotek Cytation 3, Korea).

It was confirmed that in comparison with Fc and Fc-TPP11, the binding affinity of Fc-TPP10 for the human neuropilin 1 extracellular domain at low concentration (10 nM) significantly (about 1.7-fold) increased compared to that of Fc-TPP11 (FIG. 13b).

This suggests that the regulatory T cell activity inhibitory activity of Fc-TPP11, which is induced by targeting neuropilin 1 in regulatory T cells, can more effectively occur through Fc-TPP10.

### INDUSTRIAL APPLICABILITY

The peptide that binds specifically to neuropilin 1 (NRP1) according to the present invention can inhibit the activity of regulatory T cells to inhibit the immunosuppressive response in tumor tissue, thereby down-regulating the activity thereof. Therefore, the present invention can be used for the development of a therapeutic agent against either cancer or infectious disease, such as tuberculosis, sepsis or viral hepatitis by effectively enhancing immunity of cytotoxic T cells in tumor tissue and other immunocytes.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A composition for inhibiting regulatory T cell (Treg) activity, which contains, as an active ingredient, a peptide that binds specifically to neuropilin 1 (NRP1).

2. The composition of claim 1, wherein the peptide that binds specifically to neuropilin 1 is fused to an Fc, an antibody or an antibody fragment.

3. The composition of claim 1, wherein the peptide that binds specifically to neuropilin 1 comprises one or more sequences selected from the group consisting of TPP11 (SEQ ID NO: 1), TPP1 (SEQ ID NO: 2), TPP8 (SEQ ID NO: 3), and TPP10 (SEQ ID NO: 4).

4. The composition of claim 3, wherein the peptide binding specifically to neuropilin 1 binds to an Fc, an antibody or fragment thereof by linker-mediated binding, chemical bonding, or genetic fusion.

5. The composition of claim 2, wherein the peptide binds to the C-terminus of a heavy-chain constant region (Fc) of an antibody.

6. The composition of claim 5, wherein the antibody is selected from the group consisting of IgG1, IgG2, IgG3, IgG4, IgM, IgA, IgD, and IgE.

7. The composition of claim 2, wherein the antibody is Fab, F(ab)₂, scFv, V_{H} or V_{L}.

8. The composition of claim 2, wherein the antibody or antibody fragment binds specifically to an immune checkpoint-associated antigen, a cancer cell surface-specific antigen, a tumor blood vessel-specific antigen, a tumor microenvironment-specific antigen, a regulatory T cell-specific antigen, or an antigen specific for immune cells that infiltrated into tumor tissue.

9. The composition of claim 8, wherein the antigen is selected from the group consisting of EGFR, HER2, VEGF, EpCAM, VEGFR-2, c-Met, CD20, CD19, CD22, CTLA-4, PD-1, PD-L1, TIM3, Mesothelin, TNFRSF4, PSMA, GPC3, GD2 ganglioside, CEA, FAP, integrin, and fibronectin.

10. The composition of claim 1, which inhibits cancer cell growth by: i) reducing the proportion of regulatory T cells among CD4+ T cells in tumor tissue; or ii) increasing the expression of interleukin-2 and interferon-gamma by inhibiting the activity of regulatory T cells that infiltrated into tumor tissue; or iii) increasing the growth of cytotoxic T cells by inhibiting the activity of regulatory T cells that infiltrated into tumor tissue.

11. The composition of claim 1, wherein the inhibition of regulatory T cell activity inhibits immunosuppression caused by regulatory T cells.

12. A composition for treating or preventing cancer or infectious disease, which comprises the composition of any one of claims 1 to 11.

13. The composition of claim 12, wherein the cancer is selected from the group consisting of colorectal cancer, malignant melanoma, breast cancer, ovarian cancer, head and neck cancer, pancreatic cancer, lung cancer, brain tumor, liver cancer, prostate cancer, bladder cancer, blood cancer (chronic or acute lymphocytic or lymphoblastic leukemia, chronic/acute myelogenous leukemia, etc.), ACTH-producing tumor, carcinoma of the cervix, chronic myelogenous leukemia, T-zone lymphoma, endometriosis, esophageal cancer, gallbladder cancer, Ewing's sarcoma, tongue cancer, Hodgkin's lymphoma, Kaposi's sarcoma, kidney cancer, mesothelioma, multiple myeloma, neuroblastoma, non-Hodgkin Lymphoma, osteosarcoma, ductal carcinoma in situ, penis cancer, retinoblastoma, skin cancer, gastric cancer, thyroid cancer, uterine cancer, testicular cancer, Wilms' tumor, and trophoblastoma.

14. The composition of claim 12, wherein the infectious disease is tuberculosis, sepsis or viral hepatitis.

15. A method for inhibiting the activity of regulatory T cells, which comprises a step of administering the composition of any one of claims 1 to 11 to a subject.

16. An immunomodulating agent comprising the composition of any one of claims 1 to 11.

17. The immunomodulating agent of claim 16, which further comprises an anticancer agent or which is used for coadministration with an anticancer agent.

18. An immunomodulating method, which comprises a step of administering the composition of any one of claims 1 to 11 to a subject.

19. A method for preventing or treating cancer or infectious disease, which comprises a step of administering the composition of any one of claims 1 to 11 to a subject.


<110>    AJOU UNIVERSITY INDUSTRY-ACADEMIC COOPERATION FOUNDATION

<120>    Composition for Inhibiting Activity of Regulatory T Cell
         comprising peptides which specificallly binds to Neuropilin 1

<130>    PP-B2102

<160>    14

<170>    KoPatentIn 3.0

<210>    1
<211>    14
<212>    PRT
<213>    Artificial Sequence

<220>
<223>    TPP11


<400>    1
His Thr Pro Gly Asn Ser Lys Pro Thr Arg Thr Pro Arg Arg
  1               5                   10


<210>    2
<211>    18
<212>    PRT
<213>    Artificial Sequence

<220>
<223>    TPP1


<400>    2
His Thr Pro Gly Asn Ser Asn Gln Phe Val Leu Thr Ser Thr Arg Pro
  1               5                   10                  15

Pro Arg



<210>    3
<211>    11
<212>    PRT
<213>    Artificial Sequence

<220>
<223>    TPP8


<400>    3
His Thr Pro Gly Ile Ala Thr Arg Thr Pro Arg
  1               5                   10


<210>    4
<211>    13
<212>    PRT
<213>    Artificial Sequence

<220>
<223>    TPP10

<400>    4
His Thr Pro Gly Asn Ser Lys Pro Thr Arg Thr Pro Arg
1               5                   10

<210>    5
<211>    496
<212>    PRT
<213>    Artificial Sequence

<220>
<223>    9D9-TPP11-HA

<400>    5
Met Glu Thr Asp Thr Leu Leu Leu Trp Val Leu Leu Leu Trp Val Pro
1               5                   10                  15

Gly Ser Thr Gly Asp Glu Ala Lys Leu Gln Glu Ser Gly Pro Val Leu
            20                  25                  30

Val Lys Pro Gly Ala Ser Val Lys Met Ser Cys Lys Ala Ser Gly Tyr
        35                  40                  45

Thr Phe Thr Asp Tyr Tyr Met Asn Trp Val Lys Gln Ser His Gly Lys
    50                  55                  60

Ser Leu Glu Trp Ile Gly Val Ile Asn Pro Tyr Asn Gly Asp Thr Ser
65                  70                  75                  80

Tyr Asn Gln Lys Phe Lys Gly Lys Ala Thr Leu Thr Val Asp Lys Ser
                85                  90                  95

Ser Ser Thr Ala Tyr Met Glu Leu Asn Ser Leu Thr Ser Glu Asp Ser
            100                 105                 110

Ala Val Tyr Tyr Cys Ala Arg Tyr Tyr Gly Ser Trp Phe Ala Tyr Trp
        115                 120                 125

Gly Gln Gly Thr Leu Ile Thr Val Ser Ala Lys Thr Thr Ala Pro Ser
    130                 135                 140

Val Tyr Pro Leu Ala Pro Val Cys Gly Asp Thr Thr Gly Ser Ser Val
145                 150                 155                 160

Thr Leu Gly Cys Leu Val Lys Gly Tyr Phe Pro Glu Pro Val Thr Leu
                165                 170                 175

Thr Trp Asn Ser Gly Ser Leu Ser Ser Gly Val His Thr Phe Pro Ala
            180                 185                 190

Val Leu Gln Ser Asp Leu Tyr Thr Leu Ser Ser Ser Val Thr Val Thr
        195                 200                 205

Ser Ser Thr Trp Pro Ser Gln Ser Ile Thr Cys Asn Val Ala His Pro
    210                 215                 220

Ala Ser Ser Thr Lys Val Asp Lys Lys Ile Glu Pro Arg Gly Pro Thr
225                 230                 235                 240

Ile Lys Pro Cys Pro Pro Cys Lys Cys Pro Ala Pro Asn Leu Leu Gly

245                 250                 255

Gly Pro Ser Val Phe Ile Phe Pro Pro Lys Ile Lys Asp Val Leu Met
            260                 265                 270

Ile Ser Leu Ser Pro Ile Val Thr Cys Val Val Val Asp Val Ser Glu
        275                 280                 285

Asp Asp Pro Asp Val Gln Ile Ser Trp Phe Val Asn Asn Val Glu Val
    290                 295                 300

His Thr Ala Gln Thr Gln Thr His Arg Glu Asp Tyr Asn Ser Thr Leu
305                 310                 315                 320

Arg Val Val Ser Ala Leu Pro Ile Gln His Gln Asp Trp Met Ser Gly
                325                 330                 335

Lys Glu Phe Lys Cys Lys Val Asn Asn Lys Asp Leu Pro Ala Pro Ile
            340                 345                 350

Glu Arg Thr Ile Ser Lys Pro Lys Gly Ser Val Arg Ala Pro Gln Val
        355                 360                 365

Tyr Val Leu Pro Pro Pro Glu Glu Glu Met Thr Lys Lys Gln Val Thr
    370                 375                 380

Leu Thr Cys Met Val Thr Asp Phe Met Pro Glu Asp Ile Tyr Val Glu
385                 390                 395                 400

Trp Thr Asn Asn Gly Lys Thr Glu Leu Asn Tyr Lys Asn Thr Glu Pro
                405                 410                 415

Val Leu Asp Ser Asp Gly Ser Tyr Phe Met Tyr Ser Lys Leu Arg Val
            420                 425                 430

Glu Lys Lys Asn Trp Val Glu Arg Asn Ser Tyr Ser Cys Ser Val Val
        435                 440                 445

His Glu Gly Leu His Asn His His Thr Thr Lys Ser Phe Ser Arg Thr
    450                 455                 460

Pro Gly Lys Gly Gly Gly Gly Ser Gly Gly Gly Gly Ser Gly Gly Gly
465                 470                 475                 480

Gly Ser His Thr Pro Gly Asn Ser Lys Pro Thr Arg Thr Pro Arg Arg
                485                 490                 495

<210>   6
<211>   1488
<212>   DNA
<213>   Artificial Sequence

<220>
<223>   9D9-TPP11-HD

<400>   6
atggagacag acacactcct gctatgggta ctgctgctct gggttccagg ttccactggt      60

gacgaggcaa agctgcagga gtccggaccc gtgctggtga agcctggagc cagcgtgaag     120

atgtcctgca aggcctctgg ctacaccttc acagactact atatgaactg ggtgaagcag 180

agccacggca agtccctgga gtggatcggc gtgatcaacc cctacaatgg cgacacctct 240

tataaccaga agtttaaggg caaggccacc ctgacagtgg ataagagctc ctctaccgcc 300

tacatggagc tgaatagcct gacatccgag gattctgccg tgtactattg tgcccggtac 360

tatggctctt ggttcgccta ttggggccag ggcaccctga tcacagtgag cgccaaaaca 420

acagccccat cggtctatcc actggcccct gtgtgtggag atacaactgg ctcctcggtg 480

actctaggat gcctggtcaa gggttatttc cctgagccag tgaccttgac ctggaactct 540

ggatccctgt ccagtggtgt gcacaccttc ccagctgtcc tgcagtctga cctctacacc 600

ctcagcagct cagtgactgt aacctcgagc acctggccca gccagtccat cacctgcaat 660

gtggcccacc cggcaagcag caccaaggtg gacaagaaaa ttgagcccag agggcccaca 720

atcaagccct gtcctccatg caaatgccca gcacctaacc tcttgggtgg accatccgtc 780

ttcatcttcc ctccaaagat caaggatgta ctcatgatct ccctgagccc catagtcaca 840

tgtgtggtgg tggatgtgag cgaggatgac ccagatgtcc agatcagctg gtttgtgaac 900

aacgtggaag tacacacagc tcagacacaa acccatagag aggattacaa cagtactctc 960

cgggtggtca gtgccctccc catccagcac caggactgga tgagtggcaa ggagttcaaa 1020

tgcaaggtca acaacaaaga cctcccagcg cccatcgaga gaaccatctc aaaacccaaa 1080

gggtcagtaa gagctccaca ggtatatgtc ttgcctccac cagaagaaga gatgactaag 1140

aaacaggtca ctctgacctg catggtcaca gacttcatgc ctgaagacat ttacgtggag 1200

tggaccaaca acgggaaaac agagctaaac tacaagaaca ctgaaccagt cctggactct 1260

gatggttctt acttcatgta cagcaagctg agagtggaaa agaagaactg ggtggaaaga 1320

aatagctact cctgttcagt ggtccacgag ggtctgcaca atcaccacac gactaagagc 1380

ttctcccgga ctccgggtaa aggtggagga ggatctggag gaggaggaag tggaggtgga 1440

ggatcacata ctcctggaaa tagcaaacca acacgcacac caaggcgt 1488

<210> 7
<211> 467
<212> PRT
<213> Artificial Sequence

<220>
<223> 9D9-HA

<400> 7
   Met Glu Thr Asp Thr Leu Leu Leu Trp Val Leu Leu Leu Trp Val Pro
   1 5 10 15
   Gly Ser Thr Gly Asp Glu Ala Lys Leu Gln Glu Ser Gly Pro Val Leu
   20 25 30
   Val Lys Pro Gly Ala Ser Val Lys Met Ser Cys Lys Ala Ser Gly Tyr
           35                  40                  45
   Thr Phe Thr Asp Tyr Tyr Met Asn Trp Val Lys Gln Ser His Gly Lys
       50                  55                  60
   Ser Leu Glu Trp Ile Gly Val Ile Asn Pro Tyr Asn Gly Asp Thr Ser
   65                  70                  75                  80
   Tyr Asn Gln Lys Phe Lys Gly Lys Ala Thr Leu Thr Val Asp Lys Ser
                   85                  90                  95
   Ser Ser Thr Ala Tyr Met Glu Leu Asn Ser Leu Thr Ser Glu Asp Ser
               100                 105                 110
   Ala Val Tyr Tyr Cys Ala Arg Tyr Tyr Gly Ser Trp Phe Ala Tyr Trp
           115                 120                 125
   Gly Gln Gly Thr Leu Ile Thr Val Ser Ala Lys Thr Thr Ala Pro Ser
       130                 135                 140
   Val Tyr Pro Leu Ala Pro Val Cys Gly Asp Thr Thr Gly Ser Ser Val
   145                 150                 155                 160
   Thr Leu Gly Cys Leu Val Lys Gly Tyr Phe Pro Glu Pro Val Thr Leu
                   165                 170                 175
   Thr Trp Asn Ser Gly Ser Leu Ser Ser Gly Val His Thr Phe Pro Ala
               180                 185                 190
   Val Leu Gln Ser Asp Leu Tyr Thr Leu Ser Ser Ser Val Thr Val Thr
           195                 200                 205
   Ser Ser Thr Trp Pro Ser Gln Ser Ile Thr Cys Asn Val Ala His Pro
       210                 215                 220
   Ala Ser Ser Thr Lys Val Asp Lys Lys Ile Glu Pro Arg Gly Pro Thr
   225                 230                 235                 240
   Ile Lys Pro Cys Pro Pro Cys Lys Cys Pro Ala Pro Asn Leu Leu Gly
                   245                 250                 255
   Gly Pro Ser Val Phe Ile Phe Pro Pro Lys Ile Lys Asp Val Leu Met
               260                 265                 270
   Ile Ser Leu Ser Pro Ile Val Thr Cys Val Val Val Asp Val Ser Glu
           275                 280                 285
   Asp Asp Pro Asp Val Gln Ile Ser Trp Phe Val Asn Asn Val Glu Val
       290                 295                 300
   His Thr Ala Gln Thr Gln Thr His Arg Glu Asp Tyr Asn Ser Thr Leu
   305                 310                 315                 320
   Arg Val Val Ser Ala Leu Pro Ile Gln His Gln Asp Trp Met Ser Gly
                   325                 330                 335
   Lys Glu Phe Lys Cys Lys Val Asn Asn Lys Asp Leu Pro Ala Pro Ile
               340                 345                 350
   Glu Arg Thr Ile Ser Lys Pro Lys Gly Ser Val Arg Ala Pro Gln Val
           355                 360                 365
   Tyr Val Leu Pro Pro Pro Glu Glu Glu Met Thr Lys Lys Gln Val Thr
       370                 375                 380
   Leu Thr Cys Met Val Thr Asp Phe Met Pro Glu Asp Ile Tyr Val Glu
   385                 390                 395                 400
   Trp Thr Asn Asn Gly Lys Thr Glu Leu Asn Tyr Lys Asn Thr Glu Pro
                   405                 410                 415
   Val Leu Asp Ser Asp Gly Ser Tyr Phe Met Tyr Ser Lys Leu Arg Val
               420                 425                 430
   Glu Lys Lys Asn Trp Val Glu Arg Asn Ser Tyr Ser Cys Ser Val Val
           435                 440                 445
   His Glu Gly Leu His Asn His His Thr Thr Lys Ser Phe Ser Arg Thr
       450                 455                 460
   Pro Gly Lys
   465

<210>   8
<211>   240
<212>   PRT
<213>   Artificial Sequence

<220>
<223>   9D9-LA

<400>   8
Met Glu Thr Asp Thr Leu Leu Leu Trp Val Leu Leu Leu Trp Val Pro
 1               5                  10                  15

Gly Ser Thr Gly Asp Asp Ile Val Met Thr Gln Thr Thr Leu Ser Leu
            20                  25                  30

Pro Val Ser Leu Gly Asp Gln Ala Ser Ile Ser Cys Arg Ser Ser Gln
        35                  40                  45

Ser Ile Val His Ser Asn Gly Asn Thr Tyr Leu Glu Trp Tyr Leu Gln
    50                  55                  60

Lys Pro Gly Gln Ser Pro Lys Leu Leu Ile Tyr Lys Val Ser Asn Arg
65                  70                  75                  80

Phe Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Leu Gly Val Tyr
            100                 105                 110

Tyr Cys Phe Gln Gly Ser His Val Pro Tyr Thr Phe Gly Gly Gly Thr
        115                 120                 125

Lys Leu Glu Ile Lys Arg Ala Asp Ala Ala Pro Thr Val Ser Ile Phe
    130                 135                 140

Pro Pro Ser Ser Glu Gln Leu Thr Ser Gly Gly Ala Ser Val Val Cys
145                 150                 155                 160

Phe Leu Asn Asn Phe Tyr Pro Lys Asp Ile Asn Val Lys Trp Lys Ile

                165                 170                 175

Asp Gly Ser Glu Arg Gln Asn Gly Val Leu Asn Ser Trp Thr Asp Gln
            180                 185                 190

Asp Ser Lys Asp Ser Thr Tyr Ser Met Ser Arg Thr Leu Thr Leu Thr
        195                 200                 205

Lys Asp Glu Tyr Glu Arg His Asn Ser Tyr Thr Cys Glu Ala Thr His
    210                 215                 220

Lys Thr Ser Thr Ser Pro Ile Val Lys Ser Phe Asn Arg Asn Glu Cys
225                 230                 235                 240

<210> 9
<211> 720
<212> DNA
<213> Artificial Sequence

<220>
<223> 9D9-LD

<400> 9
atggagacag acacactcct gctatgggta ctgctgctct gggttccagg ttccactggt 60

gacgacatcg tgatgaccca gaccacactg tccctgcccg tgtctctggg cgatcaggcc 120

tctatcagct gccggagctc ccagagcatc gtgcactcca acggcaatac atacctggag 180

tggtatctgc agaagcccgg ccagtcccct aagctgctga tctacaaggt gtctaaccgg 240

ttcagcggcg tgccagacag attttccggc tctggcagcg gcaccgattt cacactgaag 300

atctccaggg tggaggcaga ggacctgggc gtgtactatt gtttccaggg ctctcacgtg 360

ccctatacct ttggcggcgg cacaaagctg gagatcaagc gcgctgatgc tgcaccaact 420

gtatccatct tcccaccatc cagtgagcag ttaacatctg gaggtgcctc agtcgtgtgc 480

ttcttgaaca acttctaccc caaagacatc aatgtcaagt ggaagattga tggcagtgaa 540

cgacaaaatg gcgtcctgaa cagttggact gatcaggaca gcaaagacag cacctacagc 600

atgagcagaa ccctcacgtt gaccaaggac gagtatgaac gacataacag ctatacctgt 660

gaggccactc acaagacatc aacttcaccc attgtcaaga gcttcaacag gaatgagtgt 720

720

<210> 10
<211> 252
<212> PRT
<213> Artificial Sequence

<220>
<223> Fc(AAG)-TPP11-A

<400> 10
Thr Cys Pro Pro Cys Pro Ala Pro Glu Ala Ala Gly Gly Pro Ser Val

1               5                   10                  15

Phe Leu Phe Pro Pro Lys Pro Lys Asp Thr Leu Met Ile Ser Arg Thr
            20                  25                  30

Pro Glu Val Thr Cys Val Val Val Asp Val Ser His Glu Asp Pro Glu
        35                  40                  45

Val Lys Phe Asn Trp Tyr Val Asp Gly Val Glu Val His Asn Ala Lys
    50                  55                  60

Thr Lys Pro Arg Glu Glu Gln Tyr Asn Ser Thr Tyr Arg Val Val Ser
65                  70                  75                  80

Val Leu Thr Val Leu His Gln Asp Trp Leu Asn Gly Lys Glu Tyr Lys
                85                  90                  95

Cys Lys Val Ser Asn Lys Ala Leu Gly Ala Pro Ile Glu Lys Thr Ile
            100                 105                 110

Ser Lys Ala Lys Gly Gln Pro Arg Glu Pro Gln Val Tyr Thr Leu Pro
        115                 120                 125

Pro Ser Arg Asp Glu Leu Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Val Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Met His Glu Ala Leu
        195                 200                 205

His Asn His Tyr Thr Gln Lys Ser Leu Ser Leu Ser Pro Gly Lys Gly
    210                 215                 220

Gly Gly Gly Ser Gly Gly Gly Gly Ser Gly Gly Gly Gly Ser His Thr
225                 230                 235                 240

Pro Gly Asn Ser Lys Pro Thr Arg Thr Pro Arg Arg
                245                 250

<210>    11
<211>    756
<212>    DNA
<213>    Artificial Sequence

<220>
<223>    Fc(AAG)-TPP11-D

<400>    11
acatgcccac cgtgcccagc acctgaagcc gccgggggac cgtcagtctt cctcttcccc        60

ccaaaaccca aggacaccct catgatctcc cggacccctg aggtcacatg cgtggtggtg       120

gacgtgagcc acgaagaccc tgaggtcaag ttcaactggt acgtggacgg cgtggaggtg       180

cataatgcca agacaaagcc gcgggaggag cagtacaaca gcacgtaccg tgtggtcagc 240

gtcctcaccg tcctgcacca ggactggctg aatggcaagg agtacaagtg caaggtctcc 300

aacaaagccc tcggcgcccc catcgagaaa accatctcca aagccaaagg gcagcctcga 360

gaaccacagg tgtacaccct gcccccatcc cgggatgagc tgaccaagaa ccaggtcagc 420

ctgacctgcc tggtcaaagg cttctatccc agcgacatcg ccgtggagtg ggagagcaat 480

gggcagccgg agaacaacta caagaccacg cctcccgtgc tggactccga cggctccttc 540

ttcctctaca gcaagctcac cgtggacaag agcaggtggc agcaggggaa cgtcttctca 600

tgctccgtga tgcatgaggc tctgcacaac cactacacgc agaagagcct ctccctgtcg 660

ccgggtaaag gtggaggagg atctggagga ggaggaagtg gaggtggagg atcacatact 720

cctggaaata gcaaaccaac acgcacacca aggcgt 756

<210> 12
<211> 223
<212> PRT
<213> Artificial Sequence

<220>
<223> Fc(AAG)-A

<400> 12
Thr Cys Pro Pro Cys Pro Ala Pro Glu Ala Ala Gly Gly Pro Ser Val
1               5                   10                  15

Phe Leu Phe Pro Pro Lys Pro Lys Asp Thr Leu Met Ile Ser Arg Thr
            20                  25                  30

Pro Glu Val Thr Cys Val Val Val Asp Val Ser His Glu Asp Pro Glu
        35                  40                  45

Val Lys Phe Asn Trp Tyr Val Asp Gly Val Glu Val His Asn Ala Lys
    50                  55                  60

Thr Lys Pro Arg Glu Glu Gln Tyr Asn Ser Thr Tyr Arg Val Val Ser
65                  70                  75                  80

Val Leu Thr Val Leu His Gln Asp Trp Leu Asn Gly Lys Glu Tyr Lys
                85                  90                  95

Cys Lys Val Ser Asn Lys Ala Leu Gly Ala Pro Ile Glu Lys Thr Ile
            100                 105                 110

Ser Lys Ala Lys Gly Gln Pro Arg Glu Pro Gln Val Tyr Thr Leu Pro
        115                 120                 125

Pro Ser Arg Asp Glu Leu Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Val Leu Asp Ser

165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Met His Glu Ala Leu
        195                 200                 205

His Asn His Tyr Thr Gln Lys Ser Leu Ser Leu Ser Pro Gly Lys
    210                 215                 220

<210>    13
<211>    252
<212>    PRT
<213>    Artificial Sequence

<220>
<223>    Fc-TPP11-A

<400>    13
Thr Cys Pro Pro Cys Pro Ala Pro Glu Leu Leu Gly Gly Pro Ser Val
1               5                   10                  15

Phe Leu Phe Pro Pro Lys Pro Lys Asp Thr Leu Met Ile Ser Arg Thr
            20                  25                  30

Pro Glu Val Thr Cys Val Val Val Asp Val Ser His Glu Asp Pro Glu
        35                  40                  45

Val Lys Phe Asn Trp Tyr Val Asp Gly Val Glu Val His Asn Ala Lys
    50                  55                  60

Thr Lys Pro Arg Glu Glu Gln Tyr Asn Ser Thr Tyr Arg Val Val Ser
65                  70                  75                  80

Val Leu Thr Val Leu His Gln Asp Trp Leu Asn Gly Lys Glu Tyr Lys
                85                  90                  95

Cys Lys Val Ser Asn Lys Ala Leu Pro Ala Pro Ile Glu Lys Thr Ile
            100                 105                 110

Ser Lys Ala Lys Gly Gln Pro Arg Glu Pro Gln Val Tyr Thr Leu Pro
        115                 120                 125

Pro Ser Arg Asp Glu Leu Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Val Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Met His Glu Ala Leu
        195                 200                 205

His Asn His Tyr Thr Gln Lys Ser Leu Ser Leu Ser Pro Gly Lys Gly
    210                 215                 220

Gly Gly Gly Ser Gly Gly Gly Gly Ser Gly Gly Gly Gly Ser His Thr
225                 230                 235                 240

Pro Gly Asn Ser Lys Pro Thr Arg Thr Pro Arg Arg
                245                 250

<210>    14
<211>    223
<212>    PRT
<213>    Artificial Sequence

<220>
<223>    Fc-A

<400>    14
Thr Cys Pro Pro Cys Pro Ala Pro Glu Leu Leu Gly Gly Pro Ser Val
  1               5                  10                  15

Phe Leu Phe Pro Pro Lys Pro Lys Asp Thr Leu Met Ile Ser Arg Thr
             20                  25                  30

Pro Glu Val Thr Cys Val Val Val Asp Val Ser His Glu Asp Pro Glu
         35                  40                  45

Val Lys Phe Asn Trp Tyr Val Asp Gly Val Glu Val His Asn Ala Lys
     50                  55                  60

Thr Lys Pro Arg Glu Glu Gln Tyr Asn Ser Thr Tyr Arg Val Val Ser
 65                  70                  75                  80

Val Leu Thr Val Leu His Gln Asp Trp Leu Asn Gly Lys Glu Tyr Lys
                 85                  90                  95

Cys Lys Val Ser Asn Lys Ala Leu Pro Ala Pro Ile Glu Lys Thr Ile
            100                 105                 110

Ser Lys Ala Lys Gly Gln Pro Arg Glu Pro Gln Val Tyr Thr Leu Pro
        115                 120                 125

Pro Ser Arg Asp Glu Leu Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Val Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Met His Glu Ala Leu
        195                 200                 205

His Asn His Tyr Thr Gln Lys Ser Leu Ser Leu Ser Pro Gly Lys
    210                 215                 220